# EUROPEAN PATENT APPLICATION

(11) **EP 1 932 926 A2**
(43) Date of publication of application: **18.06.2008**
(21) Application number: 08152833.3
(22) Date of filing: 12.08.2003
(51) Int. Cl.: C12Q 1/68

(54) **Method of diagnosing ovarian endometriosis**

(30) Priority: 30.08.2002 US 407365 P; 28.02.2003 US 450920 P
(62) Divisional of application: 03795220.7
(71) Applicant: Oncotherapy Science, Inc., Kawasaki-shi, Kanagawa 213-0012 (JP)
(72) Inventor: Nakamura, Yusuke, Kanagawa 225-0011 (JP); Katagiri, Toyomasa, Tokyo 141-0022 (JP)
(74) Representative: Vossius & Partner

(57) **Abstract**

Disclosed are methods of detecting ovarian endometriosis using differentially expressed genes. Also disclosed are methods of screening compounds serving as agents for treating ovarian endometriosis, and methods of treating ovarian endometriosis and method or vaccinating a subject against ovarian endometriosis.

## Description

The present application is related to USSN 60/407,365, filed August 30, 2002, USSN 60/450,920, filed February 28, 2003, which is incorporated herein by reference.

### Technical Field

The invention relates to methods of diagnosing ovarian endometriosis.

### Background Art

Endometriosis is defined as the presence of endometrial glands and stroma outside the uterus. This gynecologic disorder occurs in approximately 14% of women of reproductive age (Rice (2002) Ann. N.Y Acad. Sci. 955: 343-352). Its most common symptoms are progressive dysmenorrhea, dyspareunia, chronic pelvic pain and infertility. Endometriosis with ovarian cysts can be diagnosed by taking pictures such as ultrasonography or magnetic resonance image (MRI), however those without ovarian cysts are difficult to diagnose without surgical procedures (Rice (2002) Ann. N.Y. Acad. Sci. 955: 343-52) since the symptoms as mentioned above are not specific to endometriosis. For example, though the concentration of CA125, one of the tumor markers for ovarian carcinoma, sometimes elevates in endometriosis sera, it is not very useful because of its low specificity (Evers et al. (1995) "Progress Management of Endometriosis" ed. Cautinho, Parthenon Publishing Groups, Carnforth, 175-84). Furthermore, a patient whose chief complaint is dysmenorrhea and shown no physical abnormality, it is difficult to distinguish endometriosis from idiopathic dysmenorrhea.

Treatment for endometriosis commonly involves surgical resection and/or medication with hormonal agents such as gonadotropin-releasing hormone (GnRH) agonists and androgens. However, since long-term medication is undesirable because of side effects such as menopausal disorders (hot flashes and stiff shoulders), genital bleeding, and bone demineralization, clinical control of endometriosis is often very difficult. Methods for treatment of the disease have also a problem with a view to high recurrence rate. It has been reported that the recurrence rate at five years after operation was about 20% (Redwine (1991) Fertil. Steril. 56: 628-34) and that at five years after medical treatment was as much as 53% (Waller and Shaw (1993) Fertil. Steril. 59: 511-5).

Various studies for endometriosis revealed little of the underlying genetic and pathophysiologic mechanisms due to following reasons; (i) epithelial cells of endometrial cysts often wither and peel off so that there is much difficulty for obtaining sufficient material; and (ii) the effect of contamination of the stromal cells is not small under the usual sampling methods. To overcome these problems, the present inventors scraped and microdissected epithelial cells from the cysts and obtained them with high purity in a forgoing study (Jimbo et al. (1997) Am. J. Pathol. 150: 1173-8). Subsequently, candidate genes as diagnostic and/or therapeutic targets for endometriosis were identified using genome-wide cDNA microarray analyses.

TFPI-2, also known as placental protein 5 (PP5), is a serine proteinase inhibitor containing 3 tandemly arranged Kunitz-type proteinase inhibitor domains, homologous to tissue factor pathway inhibitor (Sprecher et al. (1994) Proc. Natl. Acad. Sci. USA 91: 3353-7; Rao et al. (1996) Arch. Biochem. Biophys. 335: 82-92; Miyagi et al. (1994) J. Biochem. 116: 939-42). The protein is constitutively secreted by several endothelial cell types (lino et al. (1998) Arterioscler. Thromb. Vasc. Biol. 18: 40-6) in three alternatively glycosylated isoforms of 27, 31, and 33 kDa (Rao et al. (1996) Arch. Biochem. Biophys. 335: 82-92). TFPI-2 is a strong inhibitor of plasmin as well as of trypsin, chymotrypsin, plasma kallikrein, cathepsin G, factor VIIa and factor XIa, but not urokinase-type plasminogen activator (uPA), tissue plasminogen activator or thrombin (Sprecher et al. (1994) Proc. Natl. Acad. Sci. USA 91: 3353-7; Rao et al. (1995) Arch. Biochem. Biophys. 319: 55-62; Rao et al. (1995) Arch. Biochem. Biophys. 317: 311-4; Rao et al. (1995) J. Invest. Dermatol. 104: 379-83; Peterson et al. (1996) Biochemistry 35: 266-72). Quantification of this gene with cell-conditioned-medium, extracellular matrix (ECM) and cytoplasmic fractions showed that most of the inhibitors are present in the ECM (Rao et al. (1996) Arch. Biochem. Biophys. 335: 82-92). Neaud et al. have reported that TFPI-2 potentiate hepatocyte growth factor-induced invasion of hepatocellular carcinoma cells and is capable of inducing its own invasion (Neaud et al. (2000) J. Biol. Chem. 275: 35565-9), whereas it has been demonstrated that the expression of the inhibitor inversely correlates during the progression of human glioma (Rao et al. (2001) Clin. Cancer Res. 7: 570-6). Various gynecological studies about TFPI-2 were performed in terms of progression of pregnancy, but there is no report that this protein relates to the development of endometriosis.

Intelectin (ITLN) had been reported as a secretory protein which might play a role in the recognition of bacterial-specific components in the host (Rao et al. (1996) Arch. Biochem. Biophys. 335: 82-92). Today, ITLN is proven to be a human lectin, binding to galactofuranosyl residues in the presence of Ca²⁺ that recognizes bacterial arabinogalactan of *Nocardia* containing D-galactofuranosyl residues (Tsuji et al. (2001) J. Biol. Chem. 276: 23456-63). ITLN is a secretory glycoprotein consisting of 295 amino acids and N-linked oligosaccharides, and comprises a basic structural unit of a 120 kDa homotrimer in which 40 kDa polypeptides are bridged by disulfide bonds. However, little is known whether ITLN participates in the progression or maintenance of various gynecologic disorders or tumor disease.

Invasion is a characteristic feature of endometriosis. According to the transplantation theory, endometriosis develops from endometrial fragments that are retrogradely menstruated into the peritoneal cavity. In order to develop into endometriotic lesions, they have to attach to the subperitoneal space and invade by interactions with ECM proteins. The proteolytic pathway of invasion depends on the balance of a lot of components including serine proteases, such as uPA and plasmin, matrix metalloproteinases and protease inhibitors (Shapiro (1998) Curr. Opin. Cell Biol. 10: 602-8; Toi et al. (1998) Breast Cancer Res. Treat. 52: 113-24; Andreasen et al. (1997) Int. J. Cancer 72: 1-22). Although the role of ECM-associated TFPI-2 is unclear, TFPI-2 may be important in the regulation of matrix turnover by serine proteases since TFPI-2 is found primarily in ECM. Moreover, Neaud et al. reported that TFPI-2 induce an invasive activity in three different human hepatocellular carcinoma cell lines and stable transfectant (Neaud et al. (2000) J. Biol. Chem. 275: 35565-9). On the contrary, it has been demonstrated that TFPI-2 strongly inhibits the *in vitro* invasion of the highly invasive HT1080 cell line (Rao et al. (1998) Int. J. Cancer 76: 749-56) and that the expression of the inhibitor inversely correlates during the progression of human glioma (Rao et al. (2001) Clin. Cancer Res. 7: 570-6). As reported by Neaud et al., TFPI-2 may have both an indirect anti-invasive effect and a direct pro-invasive effect. Other protease inhibitors, for example, plasminogen activator inhibitor-1, also share a dual effect (Deng et al. (1996) J. Cell Biol. 134: 1563-71). The interesting phenomenon that an endometriotic cell can be metastatic and invasive though the disease being a benign disorder may be explained by the fact that TFPI-2 may have such a dual effect and controls the invasion of endometriotic cells.

The potential mechanism that ITLN is involved in the pathophysiology of endometriosis might be as follows. One hypothesis is that bacterial infection participates in the development of endometriosis. It has been reported that ITLN recognizes galactofuranosyl residues of bacteria (Tsuji et al. (2001) J. Biol. Chem. 276: 23456-63). When the infection with galactofuranosyl residue containing microorganisms causes the progression of endometriosis, the expression of ITLN may be elevated as a result of the response to the infection. Furthermore, ITLN has been known to be involved in the autoimmune response of endometriosis. Dysfunction of the immune system has been implicated in the etiology of endometriosis (Giudice et al. (1998) J. Reprod. Med. 43: 252-62; Lebovic et al. (2001) Fertil. Steril. 75: 1-10). Multiple reports describe the occurrence of autoantibodies to endometrial antigens (reviewed in Burns and Schenken (1999) Clin. Obest. Gynecol. 42: 586-610). Like ITLN, most of the autoantigens are glycoproteins. Thus, ITLN might act as an autoantigen of endometriosis. On the other hand, Lang and Yeaman reported that jacalin, a jackfruit lectin specifically binding to the Thomsen-Friedenreich antigen (Galβ1-3GalNAc), binds to various endometriosis-associated autoantigens to consequently remove antibody reactivity with these autoantigens (Lang and Yeaman (2001) J. Autoimmun. 16: 151-61). There is another possibility that ITLN, like lectin jacalin, competitively works on the autoantigens and suppresses autoimmune response in endometriosis.

Therefore, the inventors contemplated that these proteins might function as diagnostic markers for endometriosis. The expression level of TFPI-2 in placenta is the highest in normal human tissues. While TFPI-2 circulates in blood of normal men and non-pregnant women in extremely low concentrations, its level increases 40-fold to 70-fold in the plasma of pregnant women (Buztow et al. (1988) Clin. Chem. 34: 1591-3). The expression of TFPI-2 in endometrial cysts indicates the possibility that this protein is secreted in the serum of endometriosis patients as well as in that of pregnant women. If this prediction is true, TFPI-2 may serve as a better marker for diagnosis of endometriosis. ITLN has been reported to be secreted in blood. Nevertheless, ITLN is a secretory protein which is suggested to circulate in blood of patients of endometriosis. Detection of these proteins in the serum of endometriosis patients is expected to establish a new diagnostic method for the disease.

cDNA microarray technologies have enabled to obtain comprehensive profiles of gene expression in normal and malignant cells, and compare the gene expression in malignant and corresponding normal cells (Okabe et al. (2001) Cancer Res. 61:2129-37; Kitahara et al. (2001) Cancer Res. 61: 3544-9; Lin et al. (2002) Oncogene 21:4120-8; Hasegawa et al. (2002) Cancer Res. 62:7012-7). This approach enables to disclose the complex nature of cancer cells, and helps to understand the mechanism of carcinogenesis. Identification of genes that are deregulated in tumors can lead to more precise and accurate diagnosis of individual cancers, and to develop novel therapeutic targets (Bienz and Clevers (2000) Cell 103:311-20). Medical applications of microarray technologies include (i) discovery of genes that contribute to tumorigenesis, (ii) discovery of useful diagnostic biomarker(s) and novel molecular target(s) for anti-cancer agents and (iii) identification of genes involved in conferring chemosensitivity. Recently, molecules associated with development of certain types of cancers identified by microarray technologies have been clinically proven to be good targets for developing effective novel drugs for cancers. To disclose mechanisms underlying tumors from a genome-wide point of view and discover target molecules for diagnosis and development of novel therapeutic agents, the present inventors have been using a microarray of cDNAs representing 23,040 human genes to analyze expression profiles of tumors from various tissues (Okabe et al. (2001) Cancer Res. 61: 2129-37; Hasegawa S. et al. (2002) Cancer Res. 62: 7012-7; Kaneta et al. (2002) Jpn. J. Cancer Res. 93: 849-56; Kitahara et al. (2002) Neoplasia 4: 295-303; Lin et al. (2002) Oncogene 21: 4120-8; Nagayama S. et al. (2002) Cancer Res. 62: 5859-66; Okutsu et al. (2002) Mol. Cancer Ther. 1: 1035-42; Kikuchi et al. (2003) Oncogene 22: 2192-205). Through analysis of expression profiles of hepatocellular carcinomas (HCC), for example, the present inventors showed frequent up-regulation of the *VANGL1* gene in tumor cells, and demonstrated that suppressing expression of the gene with antisense-oligonucleotides can significantly decrease the growth of HCC cells and induce apoptotic cell death (Yagyu et al. (2002) Int. J. Oncol. 220: 1173-8).

Studies designed to reveal mechanisms of carcinogenesis have already facilitated identification of molecular targets for anti-tumor agents. For example, inhibitors of farnexyltransferase (FTIs) which were originally developed to inhibit the growth-signaling pathway related to Ras, whose activation depends on posttranslational farnesylation, has been effective in treating Ras-dependent tumors in animal models (He et al. (1999) Cell 99: 335-45). Clinical trials on human using a combination or anti-cancer drugs and anti-HER2 monoclonal antibody, trastuzumab, have been conducted to antagonize the proto-oncogene receptor HER2/neu; and have been achieving improved clinical response and overall survival of breast-cancer patients (Lin et al. (2001) Cancer Res. 61: 6345-9). A tyrosine kinase inhibitor, STI-571, which selectively inactivates bcr-abl fusion proteins, has been developed to treat chronic myelogenous leukemias wherein constitutive activation of ber-ab1 tyrosine kinase plays a crucial role in the transformation of leukocytes. Agents of these kinds are designed to suppress oncogenic activity of specific gene products (Fujita et al. (2001) Cancer Res. 61: 7722-6). Therefore, gene products commonly up-regulated in cancerous cells may serve as potential targets for developing novel anti-cancer agents.

It has been demonstrated that CD8+ cytotoxic T lymphocytes (CTLs) recognize epitope peptides derived from tumor-associated antigens (TAAs) presented on MHC Class I molecule, and lyse tumor cells. Since the discovery of MAGE family as the first example of TAAs, many other TAAs have been discovered using immunological approaches (Boon (1993) Int. J. Cancer 54: 177-80; Boon and van der Bruggen (1996) J. Exp. Med. 183: 725-9; van der Bruggen et al. (1991) Science 254: 1643-7; Brichard et al. (1993) J. Exp. Med. 178: 489-95; Kawakami et al. (1994) J. Exp. Med. 180: 347-52). Some of the discovered TAAs are now in the stage of clinical development as targets of immunotherapy. TAAs discovered so far include MAGE (van der Bruggen et al. (1991) Science 254: 1643-7), gp100 (Kawakami et al. (1994) J. Exp. Med. 180: 347-52), SART (Shichijo et al. (1998) J. Exp. Med. 187: 277-88) and NY-ESO-1 (Chen et al. (1997) Proc. Natl. Acad. Sci. USA 94: 1914-8). On the other hand, gene products which had been demonstrated to be specifically overexpressed in tumor cells, have been shown to be recognized as targets inducing cellular immune responses. Such gene products include p53 (Umano et al. (2001) Brit. J. Cancer 84: 1052-7), HER2/neu (Tanaka et al. (2001) Brit. J. Cancer 84: 94-9), CEA (Nukaya et al. (1999) Int. J. Cancer 80: 92-7) and so on.

In spite of significant progress in basic and clinical research concerning TAAs (Rosenbeg et al. (1998) Nature Med. 4: 321-7; Mukherji et al. (1995) Proc. Natl. Acad. Sci. USA 92: 8078-82; Hu et al. (1996) Cancer Res. 56: 2479-83), only limited number of candidate TAAs for the treatment of adenocarcinomas, including colorectal cancer, are available. TAAs abundantly expressed in cancer cells, and at the same time which expression is restricted to cancer cells would be promising candidates as immunotherapeutic targets. Further, identification of new TAAs inducing potent and specific antitumor immune responses is expected to encourage clinical use of peptide vaccination strategy in various types of cancer (Boon and can der Bruggen (1996) J. Exp. Med. 183: 725-9; van der Bruggen et al. (1991) Science 254: 1643-7; Brichard et al. (1993) J. Exp. Med. 178: 489-95; Kawakami et al. (1994) J. Exp. Med. 180: 347-52; Shichijo et al. (1998) J. Exp. Med. 187: 277-88; Chen et al. (1997) Proc. Natl. Acad. Sci. USA 94: 1914-8; Harris (1996) J. Natl. Cancer Inst. 88: 1442-5; Butterfield et al. (1999) Cancer Res. 59: 3134-42; Vissers et al. (1999) Cancer Res. 59: 5554-9; van der Burg et al. (1996) J. Immunol. 156: 3308-14; Tanaka et al. (1997) Cancer Res. 57: 4465-8; Fujie et al. (1999) Int. J. Cancer 80: 169-72; Kikuchi et al. (1999) Int. J. Cancer 81: 459-66; Oiso et al. (1999) Int. J. Cancer 81: 387-94).

It has been repeatedly reported that peptide-stimulated peripheral blood mononuclear cells (PBMCs) from certain healthy donors produce significant levels of IFN-γ in response to the peptide, but rarely exert cytotoxicity against tumor cells in an HLA-A24 or -A0201 restricted manner in 51 Cr-release assays (Kawano et al. (2000) Cancer Res. 60: 3550-8; Nishizaka et al. (2000) Cancer Res. 60: 4830-7; Tamura et al. (2001) Jpn. J. Cancer Res. 92: 762-7). However, both of HLA-A24 and HLA-A0201 are one of the popular HLA alleles in Japanese, as well as Caucasian (Date et al. (1996) Tissue Antigens 47: 93-101; Kondo et al. (1995) J. Immunol. 155: 4307-12; Kubo et al. (1994) J. Immunol. 152: 3913-24; Imanishi et al. (1992) Proceeding of the eleventh International Histocompatibility Workshop and Conference Oxford University Press, Oxford, 1065; Williams et al. (1997) Tissue Antigen 49: 129). Thus, antigenic peptides of carcinomas presented by these HLAs may be especially useful for the treatment of carcinomas among Japanese and Caucasian. Further, it is known that the induction of low-affinity CTL in vitro usually results from the use of peptide at a high concentration, generating a high level of specific peptide/MHC complexes on antigen presenting cells (APCs), which will effectively activate these CTL (Alexander-Miller et al. (1996) Proc. Natl. Acad. Sci. USA 93: 4102-7).

### Summary of the Invention

The present invention is based on the discovery that a pattern of gene expressions is correlated with ovarian endometriosis. The genes that are differentially expressed in ovarian endometriosis are collectively referred to herein as "ovarian endometriosis-associated genes", "OEX nucleic acids" or "OEX polynucleotides" and the corresponding polypeptides encoded by the genes are referred to as "OEX polypeptides" or "OEX proteins".

Accordingly, the invention features a method of diagnosing or determining a predisposition to ovarian endometriosis in a subject by determining an expression level of an ovarian endometriosis-associated gene in a subject-derived biological sample, such as tissue sample. The phrase "ovarian endometriosis-associated gene" refers to a gene that is characterized by an expression level that differs in a cell obtained from an ovarian endometrial cell compared to a normal cell. The phrase "normal cell" indicates a cell obtained from ovarian or uterine tissue but not an endimetrial cyst cell. The ovarian endometriosis-associated genes include OEX 1-242. An alteration, increase or decrease of the expression level of the gene compared to a control level of the gene indicates that the subject suffers from or is at risk of developing ovarian endometriosis. The control level may be a normal control level or an ovarian endometriosis control level.

A normal control level indicates a level of gene expression detected in a normal, healthy individual or in a population of individuals known not to be suffering from ovarian endometriosis. A control level may be a single expression pattern derived from a single reference population or may be a plurality of expression patterns. For example, the control level can be a database of expression patterns from previously tested cells. A normal healthy individual is one with no clinical symptoms of ovarian endometriosis.

An increase in the expression level of OEX-1-97 and 186-242 detected in a test sample compared to a normal control level indicates that the subject (from whom the sample was obtained) suffers from or is at risk of developing ovarian endometriosis. In contrast, a decrease in the expression level of OEX 98-185 detected in a test sample compared to a normal control level indicates that said subject suffers from or is at risk of developing ovarian endometriosis.

Alternatively, the expression level of a panel of endometriosis-associated genes in a sample is compared to an ovarian endometriosis control level of the same panel of genes. The phrase "ovarian endometriosis control level" refers to the expression profile of the endometriosis-associated genes found in a population suffering from endometriosis.

A decrease or similarity in the expression level of OEX 1-97 and 186-242 compared to an ovarian endometriosis control level indicates that the subject suffers from or is at risk of developing ovarian endometriosis. In contrast, an increase or similarity in the expression level of OEX 98-185 compared to a normal control level indicates that said subject suffers from or is at risk of developing ovarian endometriosis.

According to the present invention, the expression of a gene may be determined as being altered when its expression level increases or decreases 10%, 25%, 50% or more compared to the control level. Alternatively, the expression of a gene may be determined as being altered when its expression level increases or decreases 1, 2, 5 or more fold compared to the control level. The expression level may be determined by detecting hybridization, e.g., on an array, of an ovarian endometriosis-associated gene probe to a gene transcript in the subject-derived biological sample.

The subject-derived biological sample used in the present invention may be any sample including tissue samples obtained from a test subject, e.g., a patient known to or suspected of having ovarian endometriosis. For example, the tissue sample preferably contains an epithelial cell. Alternatively, the biological sample is an epithelial cell from an endometrial cyst.

The invention also provides an ovarian endometriosis reference expression profile of the expression level of two or more genes of OEX 1-242. Alternatively, the invention provides an ovarian endometriosis reference expression profile of the expression levels of two or more genes of OEX 1-97 and 186-242 or OEX 98-185.

The invention further provides methods of screening a compound that inhibits or enhances the expression or activity of an ovarian endometriosis-associated gene, OEX 1-242 by contacting with a test compound a test cell expressing an ovarian endometriosis-associated gene or a cell into which a vector comprising a reporter gene linked downstream of a transcriptional regulatory region of an ovarian endometriosis-associated gene has been introduced and determining the expression level of the ovarian endometriosis-associated gene. The test cell may be an epithelial cell such as those from an endometrial cyst. Compounds altering the expression level of an ovarian endometriosis-associated gene or reporter gene are expected to reduce the symptom of endometriosis. A decrease in the expression level of one or more genes of OEX 1-97 and OEX 186-242 or that of the reporter gene linked downstream of a transcriptional regulatory region of OEX 1-97 or OEX 186-242 compared to a normal control level of the gene indicates that the test compound is an inhibitor of the ovarian endometriosis-associated gene and are expected to reduce the symptom of endometriosis. Alternatively, an increase of the expression level of one or more genes of OEX 98-185 or that of the reporter gene linked downstream of a transcriptional regulatory region of OEX 98-185 compared to a normal control level of the gene indicates that the test compound is an enhancer of expression of the ovarian endometriosis-associated gene and are expected to reduce the symptom of endometriosis.

Furthermore, the present invention provides method of screening a compound that inhibits or enhances the expression of an ovarian endometriosis-associated gene, wherein a polypeptide encoded by an ovarian endometriosis-associated gene is contacted with a test compound and determining the binding activity of the compound and the gene or the biological activity of the polypeptide. Compounds altering the binding activity of the compound and the gene or the biological activity of the polypeptide are expected to reduce the symptom of endometriosis. A decrease in the binding activity with or the biological activity of one or more polypeptides encoded by OEX 1-97 and OEX 186-242 compared to a normal control level of the gene indicates that the test compound is an inhibitor of the ovarian endometriosis-associated gene and is expected to reduce the symptom of endometriosis. Alternatively, an increase of the binding activity with or the biological activity of one or more polypeptides encoded by OEX 98-185 compared to a normal control level of the gene indicates that the test compound is an enhancer of the ovarian endometriosis-associated gene and is expected to reduce the symptom of endometriosis.

The invention also provides a kit comprising a detection reagent that binds to two or more OEX nucleic acids or which binds to a gene product encoded by the nucleic acid sequences. Also provided is an array of two or more nucleic acids that bind to OEX nucleic acids.

Therapeutic methods of the present invention include methods of treating or preventing ovarian endometriosis in a subject by inhibiting the expression of a gene selected from the group of OEX 1-97 and OEX 186-242 or the activity of a polypeptide encoded by the gene. The method can be achieved, for example, by administering to the subject an antisense, short interfering RNA (siRNA), ribozyme or antibody composition.

The antisense composition reduces the expression of a specific target gene sequence. For example, the antisense composition contains a nucleotide sequence that is complementary to a coding sequence selected from the group consisting of OEX 1-97 and OEX 186-242. The siRNA composition reduces the expression of a nucleic acid sequence selected from the group consisting of OEX 1-97 and OEX 186-242. A nucleic acid-specific ribozyme composition may be constructed so as to reduce the expression of a nucleic acid sequence selected from the group consisting of OEX 1-97 and OEX 186-242.

Alternatively, the treatment or prevention of ovarian endometriosis in a subject is carried out by administering to said subject an antibody or fragment thereof that binds to a polypeptide encoded a gene selected from the group consisting of OEX 1-97 and 186-242.

The invention also includes vaccines and vaccination methods. For example, a method of treating or preventing ovarian endometriosis in a subject is carried out by administering to the subject a vaccine containing a polypeptide encoded by a nucleic acid selected from the group consisting of OEX 1-97 and 186-242 or an immunologically active fragment of such a polypeptide. An immunologically active fragment is, for example, a polypeptide that is shorter than the full-length of the naturally-occurring protein and which induces an immune response. For example, an immunologically active fragment of a length of at least 8 amino acid residues that stimulates immune cells, such as T cell or B cell, is encompassed by the immunologically active fragment of the present invention. Immune cell stimulation is measured by detecting cell proliferation, elaboration of cytokines (e.g., IL-2) or production of an antibody.

Other therapeutic methods include wherein a compound that increases the expression or activity of OEX 98-185 administered to the patient. Furthermore, ovarian endometriosis can be treated by administering a protein encoded by OEX 98-185. The protein may be directly administered to the patient or, alternatively, may be expressed *in vivo* subsequent to being introduced into the patient, for example, by administering an expression vector or host cell carrying the down-regulated marker gene of interest. Suitable mechanisms for in vivo expression of a gene of interest are known in the art.

Furthermore, the present invention provides compositions for treating or preventing ovarian endometriosis. The composition preferably comprises at least one active component selected from the group of (1) an antisense, siRNA or ribozyme against a gene selected from the group of OEX 1-97 and OEX 186-242; (2) an antibody or fragment thereof binding to a polypeptide encoded by a gene selected from the group of OEX 1-97 and OEX 186-242; (3) polynucleotide select from group consisting of OEX 98-185, or polypeptide encoded by thereof; and (4) a compound selected by any of the method of screening for a compound that alters the expression or activity of an ovarian endometriosis-associated gene of the present invention.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents and other references mentioned herein are incorporated by reference in their entirety. In case of conflict, the present specification, including definitions, will control. In addition, the materials, methods and examples are illustrative only and not intended to be limiting.

Other features and advantages of the invention will be apparent from the following detailed description and from the claims.

### Brief Description of the Drawings

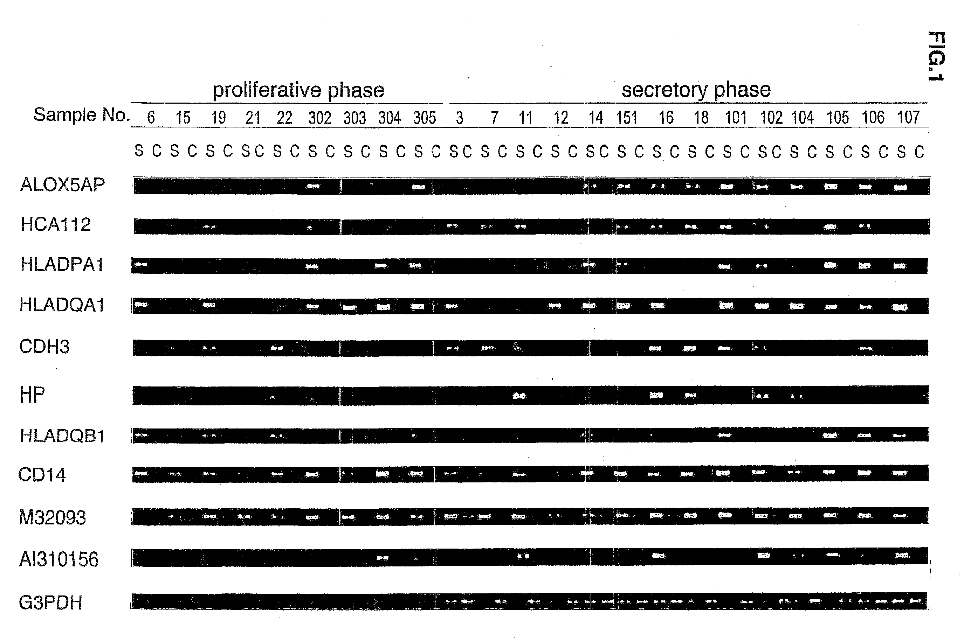

Figure 1 depicts a photograph showing the results of cDNA microarray analysis. Expression of representative 10 genes and G3PDH was examined by semi-quantitative RT-PCR using cDNA prepared from amplified RNA. S, sample; and C, control. Symbols for genes whose function was known or inferred are noted; and Accession No. for ESTs.

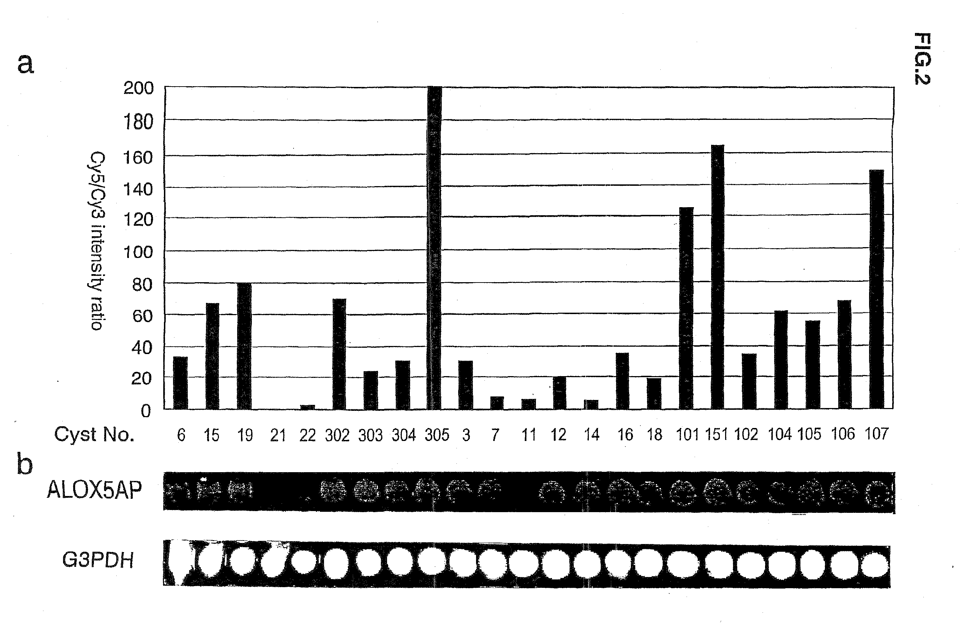

Figure 2a depicts a bar graph showing the Cy5/Cy3 intensity ratios of ALOX5AP in 23 endometrial cysts. The ratio was greater than 2.0 in all samples except No. 21.
Figure 2b depicts a photograph showing the results of gene expression assay. The expression of ALOX5AP was up-regulated. G3PDH was used as an internal control.

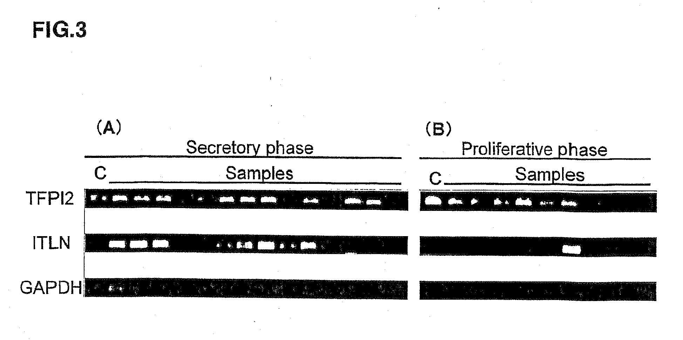

Figure 3 depicts the expression of TFPI-2 and ITLN in endometriosis. The expression of TFPI-2, ITLN and G3PDH was examined by semi-quantitative RT-PCR using cDNA prepared from amplified RNA. C, control. (A)The expression of TFPI-2 was up-regulated in 9 of 14 cases in the secretory phase; (B) the expression of ITLN was up-regulated in 10 of 14 cases in the secretory phase and in 4 of 9 cases in the proliferative phase.

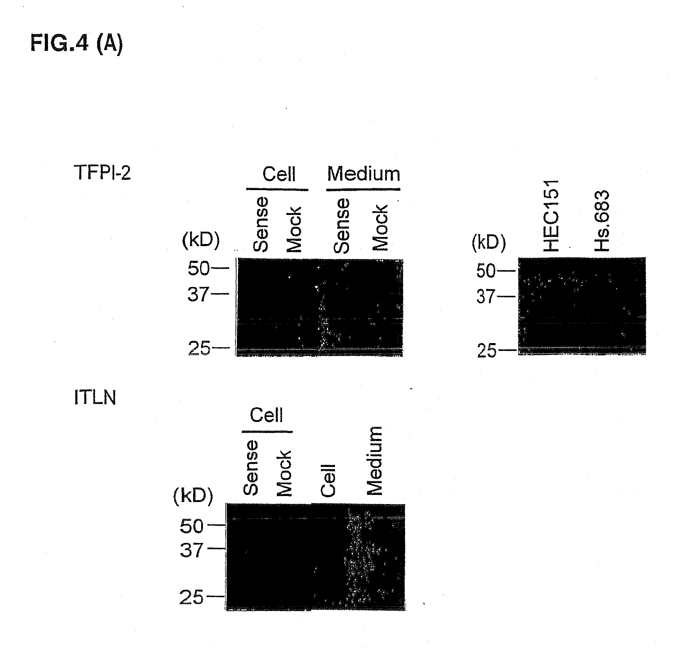

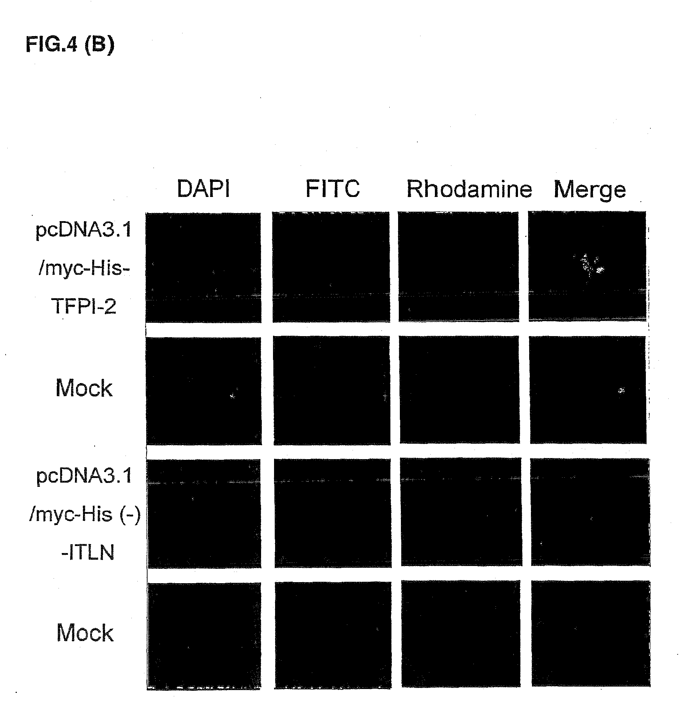

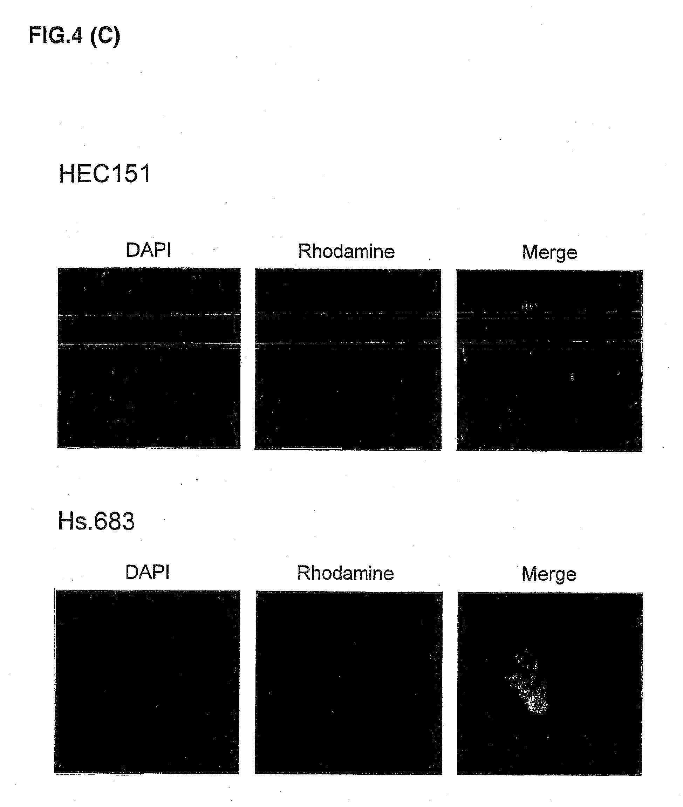

Figure 4 depicts the sub-cellular localization of TFPI-2 and ITLN proteins. (A) shows the result of Western blot analysis of myc-tagged TFPI-2 and ITLN proteins using extracts of COS-7 cells transfected with pcDNA3.1-myc/His-sense plasmid or mock. Cell, cell lysate; and Medium, conditioned medium. Each conditioned medium included the corresponding protein. In addition, endogenous expression of TFPI-2 was detected in HEC-151 and Hs.683 cells. (B) shows the result of immunofluorescent staining of TFPI-2-, ITLN- and Mock- stable transfectants. (C) shows the result of immunofluorescent staining of HEC-151 and Hs. 683 cells with anti-TFPI-2 antibody. Both exogenous and endogenous expression patterns of TFPI-2 were identical.

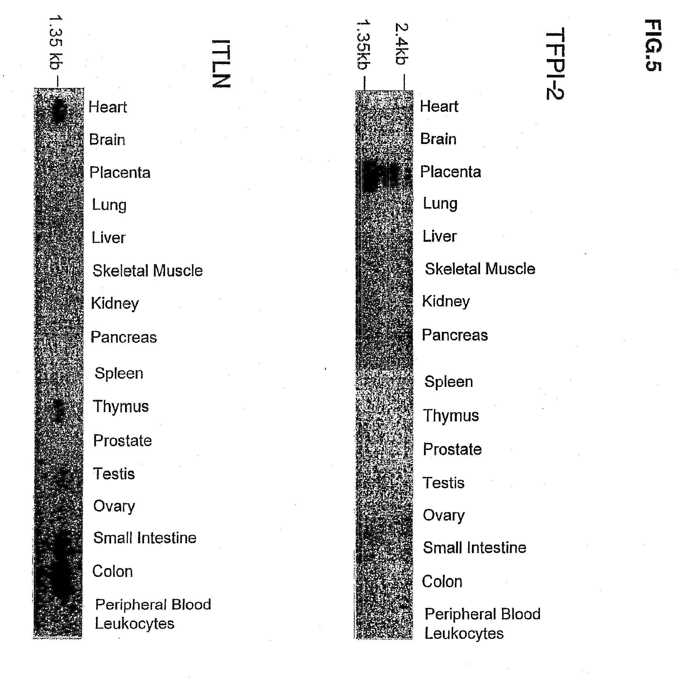

Figure 5 depicts the results of Northern blot analyses of TFPI-2 and ITLN mRNAs. The Multiple-tissue Northern blot membranes (Clontech) were hybridized with the cDNA fragments of TFPI-2 or ITLN as described under "Materials and methods". RNA size markers are indicated in kilobase pairs (kb).

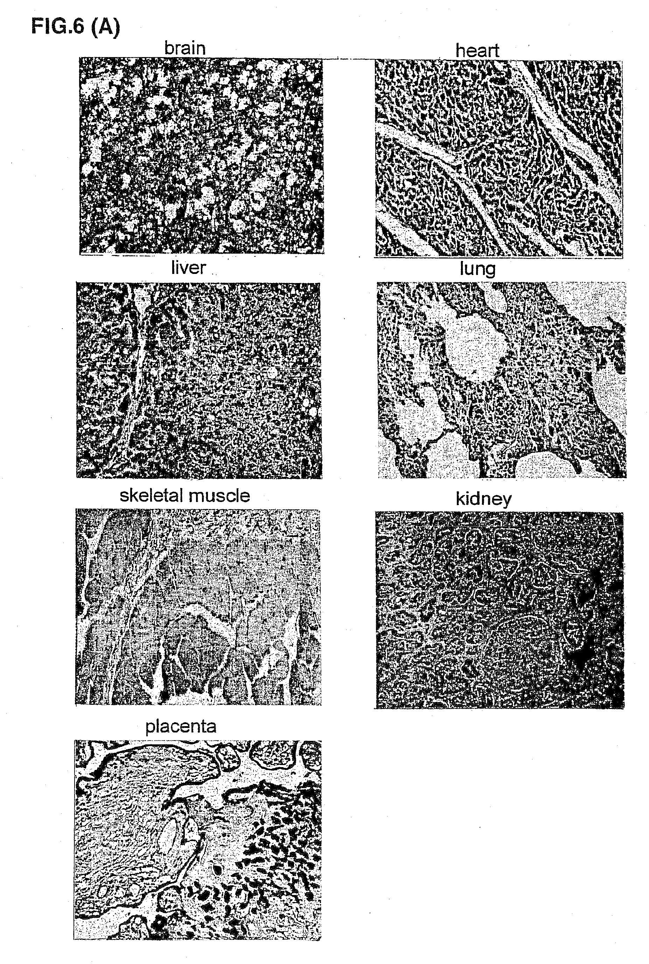

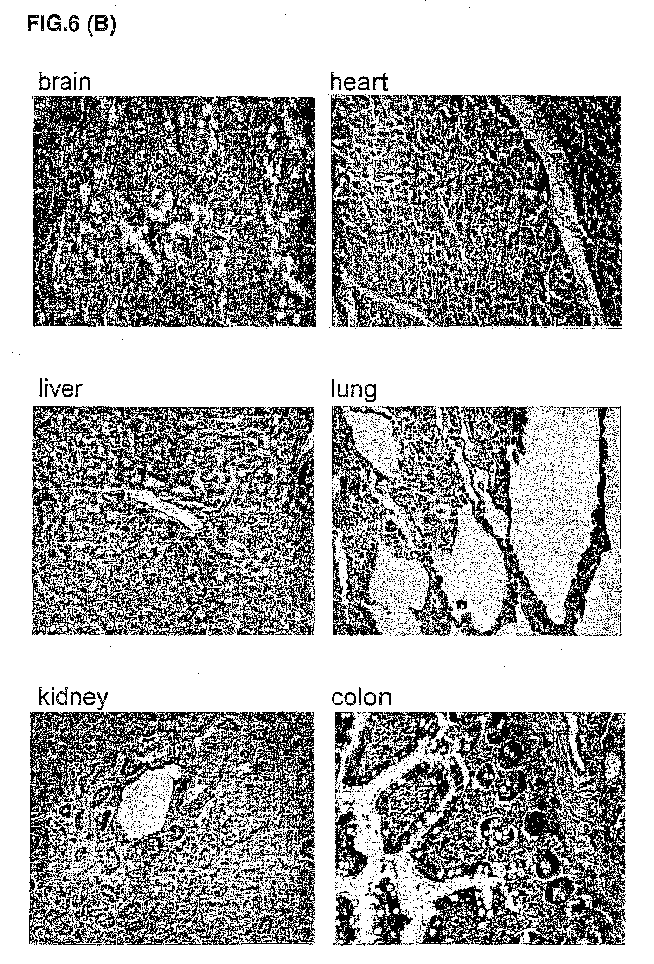

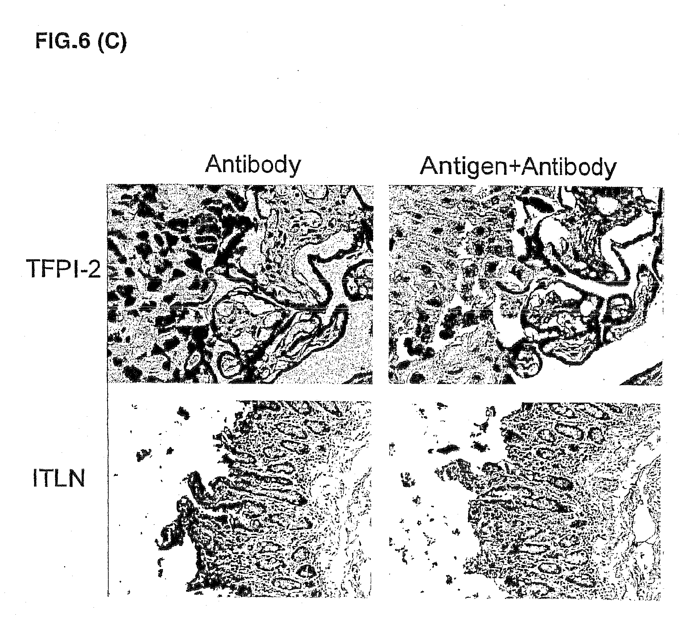

Figure 6 depicts the result of immunohistochemical staining of TFPI-2 and ITLN proteins. (A) shows the result of imunohistochemical staining of human adult normal tissue sections with anti-TFPI-2 antibody. Strong staining was observed in placenta, a much weaker staining in liver and heart, and no staining in brain, kidney, lung and skeletal muscle. (B) shows the result of immunohistochemical staining of human adult normal tissue sections with anti-ITLN antibody. Positive staining was observed in colon, much weaker in heart, and no staining in brain, kidney, liver and lung. (C) shows the result of cross- inhibition assay. Recombinant TFPI-2 protein inhibited TFPI-2 staining in placenta (upper panels). Similarly, recombinant ITLN protein inhibited ITLN staining in small intestine (lower panels).

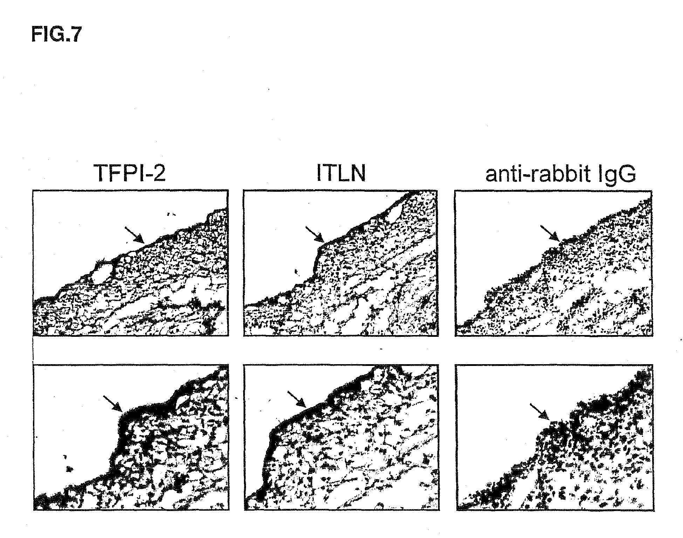

Figure 7 depicts the expression pattern of TFPI-2 and ITLN proteins in endometrial cysts examined by immunohistochemistry. Strong staining was observed on epithelial cells, and to some extent, on stromal cells of endometrial cysts by anti-TFPI-2 antibody (left panels). On the other hand, positive staining was observed only on epithelial cells of the cysts by anti-ITLN antibody (middle panels). No positive staining could be observed in the same endometrial cysts tissues with anti-rabbit IgG as the negative control (right panels). Upper panels are lower magnification images (X100) and lower panels are higher magnification ones (X200). Arrows indicate epithelial cells of the cysts.

### Detailed Description of the Invention

The words "a", "an", and "the" as used herein mean "at least one" unless otherwise specifically indicated.

The present invention is based in part on the discovery of changes in expression patterns of multiple nucleic acid sequences in endothelial cells of endometrial cysts of patients with ovarian endometriosis. The difference in gene expression was identified using a comprehensive cDNA microarray system.

To elucidate the nature of endometriosis, the present inventors carried out cDNA microarray analyses as described previously (Arimoto et al. (2003) Int. J. Oncol. 22: 551-60). By comparing the expression patterns between endometriotic tissues and corresponding eutopic endometria, several genes that were commonly up-regulated (over-expressed) in the endometrial cysts were identified. Among the identified genes, two genes encoding tissue factor pathway inhibitor-2 (TFPI-2) and intelectin (ITLN) were further examined. These two genes were selected in the present study because; (i) they showed a relatively lower expression in normal human vital organ tissues (when genes with high expression levels in organisms required for the maintenance of life are used as the target for drugs, fatal side effects may be induced); and (ii) both TFPI-2 and ITLN are known as secreted proteins and specific antibodies to these proteins can be used for diagnosis and/or treatment of diseases related to these genes.

In this study, the present inventors report that expression of these genes might play important roles in the progression or maintenance of endometriosis, and suggest that these gene products might be promising targets for the development of drugs for endometriosis.

cDNA microarray analysis was performed on over 20,000 genes. As a result, genes that were commonly over-expressed or suppressed (down-regulated or underexpressed) among ovarian endometriosis patients were selected. Two hundred forty-two genes were found to be differentially expressed in epithelial cells from endometrial cysts. Twenty-four genes were up-regulated throughout the menstrual cycle and thirty were down-regulated throughout the menstrual cycle. Seventy genes and ESTs were up-regulated during the proliferative phase of the menstrual cycle, and fourteen genes were down-regulated during the proliferative phase of the menstrual cycle. Sixty genes and ESTs were up-regulated during the secretory phase of the menstrual cycle, and forty-four genes were down-regulated during the proliferative phase of the menstrual cycle.

Hitherto, several hormonal therapies have been applied to the treatment of endometriosis. Endometriosis with ovarian chocolate cysts or severe adhesion is often treated by laparoscopic or abdominal surgery as well. However, these treatments are burdened by high recurrence rates and side effects. The differentially expressed genes identified herein may be used for diagnostic purposes and to develop gene targeted therapeutic approaches for inhibiting endometriosis and malignancies of ovarian and uterine tissue.

The genes whose expression levels are modulated *(i.e.,* increased or decreased) in ovarian endometriosis patients are summarized in Tables 1-9 and are collectively referred to herein as "ovarian endometriosis-associated genes", "OEX nucleic acids" or "OEX polynucleotides" and the corresponding polypeptides encoded by the genes are referred to as "OEX polypeptides" or "OEX proteins". Unless indicated otherwise, "OEX" refers to any of the sequences disclosed herein (OEX 1-242). The genes have been previously described and are presented along with a database accession number.

By measuring expression of the various genes in a sample comprising a cell or population of cells, ovarian endometriosis can be diagnosed in a patient. Similarly, by measuring the expression of these genes in response to various agents, agents for treating ovarian endometriosis can be identified.

**Table 1. Genes up-regulated in endometrial cysts throughout the menstrual cycle**

| ratio≥ 2 | GenBank Accession No. | Abbreviation | Gene name | OEX Assignment |
|---|---|---|---|---|
| 22 | M63262 | ALOX5AP | arachidonate 5-lipoxygenase-activating protein | 1 |
| 22 | AA583491 | HCA112 | hepatocellular carcinoma-associated antigen 112 | 2 |
| 21 | X00457 | HLA-DPA1 | major histocompatibility complex, class II, DP alpha 1 | 3 |
| 20 | M77349 | TGFBI | transforming growth factor, beta-induced, 68kD | 4 |
| 20 | K01505 | HLA-DQA1 | major histocompatibility complex, class II, DQ alpha 1 | 5 |
| 20 | X63629 | CDH3 | cadherin 3, type 1, P-cadherin (placental) | 6 |
| 20 | D85376 | | Human DNA for thyrotropin-releasing hormone receptor, exon 3 and complete cds | 7 |
| 19 | K01171 | HLA-DRA | major histocompatibility complex, class II, DR alpha | 8 |
| 18 | X00637 | HP | haptoglobin | 9 |
| 18 | M81141 | HLA-DQB1 | major histocompatibility complex, class II, DQ beta 1 | 10 |
| 18 | M86511 | CD14 | CD 14 antigen | 11 |
| 18 | M15178 | HLA-DRB1 | major histocompatibility complex, class II, DR beta 1 | 12 |
| 17 | U70136 | PRG4 | proteoglycan 4, (megakaryocyte stimulating factor, articular superficial zone protein) | 13 |
| Other genes | | | | |
| 21 | M32093 | | ESTs | 14 |
| 17 | AI310156 | | ESTs, Weakly similar to A4P_HUMAN INTESTINAL MEMBRANE A4 PROTEIN [H.sapiens] | 15 |

Genes with normalized expression ratios (cyst/normal) of ≥2.0 in more than 70% of the 23 cases were examined. The numbers of samples in that category are indicated in the left-most column. Accession numbers, gene symbols and names were retrieved from the Unigene Database (build#131).

**Table 2. Genes up-regulated in endometrial cysts from patients in the proliferative phase of the menstrual cycle**

| ratio ≥2 | Genbank Accession No. | Abbreviation | Gene name | OEX Assignment |
|---|---|---|---|---|
| 9 | AA548449 | TPM1 | tropomyosin 1 (alpha) | 16 |
| 9 | AA778308 | RNASE 1 | ribonuclease, RNase A family, 1 (pancreatic) | 17 |
| 8 | AA985222 | CTSB | cathepsin B | 18 |
| 8 | AA779820 | RBP1 | retinol-binding protein 1, cellular | 19 |
| 8 | AF034374 | | molybdenum cofactor biosynthesis protein A; molybdenum cofactor biosynthesis protein C | 20 |
| 8 | M83202 | LTF | lactotransferrin | 21 |
| 8 | H52870 | CDC10 | CDC10 (cell division cycle 10, S. cerevisiae, homolog) | 22 |
| 8 | X06617 | RPS11 | ribosomal protein S11 | 23 |
| 8 | AF023462 | PHYH | phytanoyl-CoA hydroxylase (Refsum disease) | 24 |
| 8 | AI287963 | PRP8 | U5 snRNP-specific protein (220 kD), ortholog of S. cerevisiae Prp8p | 25 |
| 8 | X99920 | S100A13 | S100 calcium-binding protein A13 | 26 |
| 7 | V00478 | ACTB | actin, beta | 27 |
| 7 | U44403 | SLA | Src-like-adapter | 28 |
| 7 | U90913 | TIP 1 | Tax interaction protein 1 | 29 |
| 7 | Y13287 | GDI2 | GDP dissociation inhibitor 2 | 30 |
| 7 | M55513 | KCNA5 | potassium voltage-gated channel, shaker-related subfamily, member 5 | 31 |
| 7 | AA627679 | CHN2 | chimerin (chimaerin) 2 | 32 |
| 7 | AA921313 | RPT.11 | ribosomal protein L11 | 33 |
| 7 | L12535 | RSU1 | Ras suppressor protein 1 | 34 |
| 7 | J04130 | SCYA4 | small inducible cytokine A4 (homologous to mouse Mipb) | 35 |
| 7 | AA961412 | UBA52 | ubiquitin A-52 residue ribosomal protein fusion product 1 | 36 |
| 7 | AA399392 | VPS11 | vacuolar protein sorting 11 (yeast homolog) | 37 |
| 7 | D 16469 | ATP6S1 | ATPase, H+ transporting, lysosomal (vacuolar proton pump), subunit 1 | 38 |
| 7 | AF006084 | ARPC1B | actin related protein 2/3 complex, subunit 1A (41 kD) | 39 |
| 7 | X99209 | HRMT1L1 | HMT1 (hnRNP methyltransferase, S. cerevisiae)-like 1 | 40 |
| 7 | AA434323 | P5CR2 | pyrroline 5-carboxylate reductase isoform | 41 |
| 7 | J02854 | MYRL2 | myosin regulatory light chain 2, smooth muscle isoform | 42 |
| Other genes | | | | |
| 8 | AI333234 | | hypothetical protein | 43 |
| 8 | AA703807 | | ESTs | 44 |
| 8 | R61506 | LOC51303 | FK506 binding protein precursor | 45 |
| 7 | H40445 | | ESTs, Weakly similar to pro alpha 1(I) collagen [H.sapiens] | 46 |
| 7 | H12942 | | ESTs | 47 |
| 7 | AI038441 | | ESTs | 48 |
| 7 | AA994249 | | ESTs | 49 |
| 7 | AI242789 | | ESTs | 50 |
| 7 | AI222007 | | ESTs | 51 |
| 7 | AA921763 | | ESTs | 52 |
| 7 | BE894625 | | Homo sapiens cDNA clone IMAGE:3918395 5' mRNA sequence | 53 |
| 7 | AA421326 | | Homo sapiens cDNA: FLJ21918 fis, clone HEP04006 | 54 |
| 7 | K00627 | | Human kpni repeat mRNA (cDNA clone pcd-kpni-8), 3' end | 55 |
| 7 | W79221 | PTD009 | PTD009 protein | 56 |
| 7 | AW513042 | KIAA1169 | two-pore channel 1, homolog | 57 |

Genes with normalized expression ratios (cyst/normal) of ≥2.0 in more than 70% of the nine cases were examined. The left-most column indicates the numbers of samples in that category. Accession numbers, gene symbols and names were retrieved from the Unigene Database (build#131).

**Table 3. Genes up-regulated in endometrial cysts only during the secretory phase of the menstrual cycle**

| ratio ≥2 | Genbank Accession No. | Abbreviation | Gene name | OEX Assignment |
|---|---|---|---|---|
| 14 | W45244 | C3 | complement component 3 | 58 |
| 13 | D28124 | NBL1 | neuroblastoma, suppression of tumorigenicity 1 | 59 |
| 13 | AA319695 | CEBPD | CCAAT/enhancer binding protein (C/EBP), delta | 60 |
| 13 | X67698 | HE1 | epididymal secretory protein (19.5kD) | 61 |
| 13 | S67310 | BF | B-factor, properdin | 62 |
| 13 | L42176 | FHL2 | four and a half LIM domains 2 | 63 |
| 12 | J04080 | C1S | complement component 1, s subcomponent | 64 |
| 12 | AA593793 | HEBP | heme-binding protein | 65 |
| 12 | U31525 | GYG | glycogenin | 66 |
| 12 | M63959 | LRPAP1 | low density lipoprotein-related protein-associated protein 1 (alpha-2-macroglobulin receptor-associated protein 1) | 67 |
| 12 | X04701 | C1R | complement component 1, r subcomponent | 68 |
| 12 | U44772 | PPT1 | palmitoyl-protein thioesterase 1 (ceroid-lipofuscinosis, neuronal 1, infantile) | 69 |
| 12 | N27409 | RPS23 | ribosomal protein S23 | 70 |
| 11 | M29877 | FUCA1 | fucosidase, alpha-L- 1, tissue | 71 |
| 11 | K03000 | ALDH1 | aldehyde dehydrogenase 1, soluble | 72 |
| 11 | AI061385 | SC5DL | sterol-C5-desaturase (fungal ERG3, delta-5-desaturase)-like | 73 |
| 11 | X04481 | C2 | complement component 2 | 74 |
| 11 | AA682870 | CCND2 | cyclin D2 | 75 |
| 11 | AF055066 | HLA-F | major histocompatibility complex, class I, F | 76 |
| 11 | AU155489 | MMP7 | matrix metalloproteinase 7 (matrilysin, uterine) | 77 |
| 10 | D78014 | DPYSL3 | dihydropyrimidinase-like 3 | 78 |
| 10 | AA704399 | UBE2N | ubiquitin-conjugating enzyme E2N (homologous to yeast UBC13) | 79 |
| 10 | D55654 | MDH1 | malate dehydrogenase 1, NAD (soluble) | 80 |
| 10 | AI985921 | CAV1 | caveolin 1, caveolae protein, 22kD | 81 |
| 10 | AA809819 | CREG | cellular repressor of E1A-stimulated genes | 82 |
| 10 | AA777014 | DAB2 | disabled (Drosophila) homolog 2 (mitogen-responsive phosphoprotein) | 83 |
| 10 | U07231 | GRSF1 | G-rich RNA sequence binding factor 1 | 84 |
| 10 | L13210 | LGALS3BP | lectin, galactoside-binding, soluble, 3 binding protein (galectin 6 binding protein) | 85 |
| 10 | D87258 | PRSS11 | protease, serine, 11 (IGF binding) | 86 |
| 10 | AA432312 | TSPYL | TSPY-like | 87 |
| ESTs and genes with unknown function | | | | |
| 12 | BF593563 | DKFZP564A 2416 | DKFZP564A2416 protein | 88 |
| 12 | AI142828 | | Homo sapiens adlican mRNA, complete cds | 89 |
| 12 | AI185130 | KIAA0193 | KIAA0193 gene product | 90 |
| 11 | N70341 | KIAA06 72 | ESTs | 91 |
| 11 | AA665097 | LOC51323 | hypothetical protein | 92 |
| 10 | D87465 | KIAA0275 | KIAA0275 gene product | 93 |
| 10 | Z25391 | KIAA0728 | KIAA0728 protein | 94 |
| 10 | AA447864 | KIAA1055 | KIAA1055 protein | 95 |
| 10 | AI343963 | PP2135 | PP2135 protein | 96 |
| 10 | AI366597 | | ESTs | 97 |

Genes with normalized expression ratios (cyst/normal) of ≥2.0 in more than 70% of the 14 case were examined. The left-most column indicates the numbers of samples in that category. Accession numbers, gene symbols and names were retrieved from the Unigene Database (build#131).

**Table 4. Genes down-regulated in endometrial cysts throughout the menstrual cycle**

| ratio ≤0.3 | Genbank Accession No. | Abbreviation | Gene name | OEX Assignment |
|---|---|---|---|---|
| 21 | D86724 | ARG2 | arginase, type II | 98 |
| 21 | D87116 | MAP2K3 | mitogen-activated protein kinase kinase 3 | 99 |
| 20 | AI339572 | KLF5 | Kruppel-like factor 5 (intestinal) | 100 |
| 20 | U19906 | AVPR1A | arginine vasopressin receptor 1A | 101 |
| 20 | Y10032 | SGK | serum/glucocorticoid regulated kinase | 102 |
| 20 | M68891 | GATA2 | GATA-binding protein 2 | 103 |
| 19 | AA121949 | BAG3 | BCL2-associated athanogene 3 | 104 |
| 19 | U83981 | GADD34 | growth arrest and DNA-damage-inducible 34 | 105 |
| 19 | X85133 | RBBP6 | retinoblastoma-binding protein 6 | 106 |
| 19 | D86956 | HSP105B | heat shock 105kD | 107 |
| 19 | W20076 | NXF1 | nuclear RNA export factor 1 | 108 |
| 19 | AI367368 | FACL5 | long-chain fatty acid coenzyme A ligase 5 | 109 |
| 18 | L17131 | HMGIY | high-mobility group (nonhistone chromosomal) protein isoforms I and Y | 110 |
| 18 | U09550 | OVGP1 | oviductal glycoprotein 1, 120kD (mucin 9, oviductin) | 111 |
| 18 | L16876 | CYP2C18 | cytochrome P-450 2C18 | 112 |
| 18 | U73843 | ELF3 | E74-like factor 3 (ets domain transcription factor, epithelial-specific) | 113 |
| 18 | AI014398 | RASD1 | RAS, dexamethason-induced 1 | 114 |
| 17 | AA436509 | IER5 | Immediate early response 5 | 115 |
| 17 | X15729 | DDX5 | DEAD/H (Asp-Glu-Ala-Asp/His) box polypeptide 5 (RNA helicase, 68kD) | 116 |
| 17 | D 13388 | HSJ2 | heat shock protein, DNAJ-like 2 | 117 |
| 17 | D90070 | PMAIP1 | phorbol2-myristate3-acetate-induced protein 1 | 118 |
| 17 | D49547 | DNAJB1 | DnaJ (Hsp40) homolog, subfamily B, member 1 | 119 |
| 17 | M16441 | LTA | lymphotoxin alpha (TNF superfamily, member 1) | 120 |
| 17 | U91618 | NTS | neurotensin | 121 |
| 17 | L13943 | GK | glycerol kinase | 122 |
| 17 | U63329 | MUTYH | mutY (E. coli) homolog | 123 |
| 17 | U40462 | ZNFN1A1 | zinc finger protein, subfamily 1A, 1 (Ikaros) | 124 |
| 17 | AA234506 | LRRFIP1 | leucine rich repeat (in FLII) interacting protein 1 | 125 |
| 17 | AI249000 | LIM | LIM protein (similar to rat protein kinase C-binding enigma) | 126 |
| 17 | AA496218 | STAG2 | stromal antigen 2 | 127 |

Genes with normalized expression ratios (cyst/normal) of ≤0.3 in more than 70% of the 23 cases were examined. The left-most column indicates the numbers of samples in that category. Accession numbers, gene symbols and names were retrieved from the Unigene Database (build#131).

**Table 5. Genes down-regulated in endometrial cysts only during the proliferative phase of the menstrual cycle**

| ratio ≤0.3 | GenBank Accession No. | Abbreviation | Gene name | OEX Assignment |
|---|---|---|---|---|
| 9 | D86955 | SCYA20 | small inducible cytokine subfamily A (Cys-Cys), member 20 | 128 |
| 9 | D87953 | NDRG1 | N-myc downstream regulated | 129 |
| 8 | AF037335 | CA12 | carbonic anhydrase XII | 130 |
| 8 | D37766 | LAMB3 | laminin, beta 3 (nicein (125kD), kalinin (140kD), BM600 (125kD)) | 131 |
| 8 | AA053789 | ZNF216 | zinc finger protein 216 | 132 |
| 8 | U62015 | CYR61 | cysteine-rich, angiogenic inducer, 61 | 133 |
| 8 | U67784 | RDC1 | G protein-coupled receptor | 134 |
| 7 | AI081684 | VNN1 | vanin 1 | 135 |
| 7 | X97324 | ADFP | adipose differentiation-related protein | 136 |
| 7 | AA634090 | HNRPA1 | heterogeneous nuclear ribonucleoprotein A1 | 137 |
| 7 | U25997 | STC1 | stanniocalcin 1 | 138 |
| 7 | AA977557 | GOLPH2 | golgi membrane protein GP73 | 139 |
| 7 | Z46629 | SOX9 | SRY (sex determining region Y)-box 9 (campomelic dysplasia, autosomal sex-reversal) | 140 |
| 7 | AB001636 | DDX15 | DEAD/H (Asp-Glu-Ala-Asp/His) box polypeptide 15 | 141 |

Genes with normalized expression ratios (cyst/normal) of ≤0.3 in more than 70% of the nine cases were examined. The left-most column indicates the numbers of samples in that category. Accession numbers, gene symbols and names were retrieved from the Unigene Database (build#131).

**Table 6. Genes down-regulated in endometrial cysts only during the secretory phase of the menstrual cycle**

| ratio ≤0.3 | GenBank Accession No. | Abbreviation | Gene name | OEX Assignment |
|---|---|---|---|---|
| 14 | AI298111 | MRPS2 | mitochondrial ribosomal protein S2 | 142 |
| 14 | AA534943 | SCYB14 | small inducible cytokine subfamily B (Cys-X-Cys), member 14 (BRAK) | 143 |
| 13 | X58295 | GPX3 | glutathione peroxidase 3 (plasma) | 144 |
| 13 | AA565113 | DRPLA | dentatorubral-pallidoluysian atrophy (atrophin) | 145 |
| 12 | T84015 | PLEC1 | plectin 1, intermediate filament binding protein, 500kD | 146 |
| 12 | AA279817 | GADD45B | growth arrest and DNA-damage-inducible, beta | 147 |
| 12 | AI160184 | LOC51673 | brain specific protein | 148 |
| 12 | AW162122 | APC4 | anaphase-promoting complex subunit 4 | 149 |
| 12 | X03438 | CSF3 | colony stimulating factor 3 (granulocyte) | 150 |
| 12 | AF007162 | CRYAB | crystallin, alpha B | 151 |
| 12 | M60974 | GADD45A | growth arrest and DNA-damage-inducible, alpha | 152 |
| 12 | X03473 | H1F0 | H1 histone family, member 0 | 153 |
| 12 | M69226 | MAOA | monoamine oxidase A | 154 |
| 12 | M95548 | SLC3Al | solute carrier family 3 (cystine, dibasic and neutral amino acid transporters, activator of cystine, dibasic and neutral amino acid transport), member 1 | 155 |
| 12 | AI349114 | TCTE1L | t-complex-associated-testis-expressed 1-like | 156 |
| 11 | AA639795 | FRSB | phenylalanyl-tRNA synthetase beta-subunit | 157 |
| 11 | AF073710 | RGS9 | regulator of G-protein signalling 9 | 158 |
| 11 | AF039691 | HDAC5 | histone deacetylase 5 | 159 |
| 11 | L34059 | CDH4 | cadherin 4, type 1, R-cadherin (retinal) | 160 |
| 11 | D10922 | FPRL1 | formyl peptide receptor-like 1 | 161 |
| 11 | Y10313 | IFRD1 | interferon-related developmental regulator 1 | 162 |
| 11 | AI186556 | PISD | phosphatidylserine decarboxylase | 163 |
| 11 | L26260 | STK19 | serine/threonine kinase 19 | 164 |
| 11 | AA419482 | LOC54518 | similar to proline-rich protein 48 | 165 |
| 11 | L01100 | ICA1 | islet cell autoantigen 1 (69kD) | 166 |
| 11 | AF014398 | IMPA2 | inositol(myo)(or 4)-monophosphatase 2 | 167 |
| 11 | AA676322 | MTF1 | metal-regulatory transcription factor 1 | 168 |
| 10 | AI126155 | CUL3 | cullin 3 | 169 |
| 10 | M11717 | HSPA1A | heat shock 70kD protein 1A | 170 |
| 10 | U08015 | NFATC1 | nuclear factor of activated T-cells, cytoplasmic, calcineurin-dependent 1 | 171 |
| 10 | AA608780 | GKP2 | Glycerol kinase pseudogene 2 | 172 |
| 10 | D16581 | NUDT1 | nudix (nucleoside diphosphate linked moiety X)-type motif 1 | 173 |
| 10 | AF066859 | PYGM | phosphorylase, glycogen; muscle (McArdle syndrome, glycogen storage disease type V) | 174 |
| 10 | AA312113 | RBL1 | retinoblastoma-like 1 (p107) | 175 |
| 10 | AA912674 | JAM2 | vascular endothelial junction-associated molecule | 176 |
| 10 | AI261581 | AGPS | alkylglycerone phosphate synthase | 177 |
| 10 | M78798 | CERD4 | Cer-d4 (mouse) homolog | 178 |
| 10 | M31452 | C4BPA | complement component 4-binding protein, alpha | 179 |
| 10 | U48734 | ACTN4 | actinin, alpha 4 | 180 |
| 10 | M16451 | CKB | creatine kinase, brain | 181 |
| 10 | M63582 | TRH | thyrotropin-releasing hormone | 182 |
| 10 | X16940 | ACTG2 | actin, gamma 2, smooth muscle, enteric | 183 |
| 10 | AI278397 | DLX5 | distal-less homeo box 5 | 184 |
| 10 | N70019 | MT1E | metallothionein 1E (functional) | 185 |

Genes with normalized expression ratios (cyst/normal) of ≤0.3 in more than 70% of the 14 cases were examined. The left-most column indicates the numbers of samples in that category. Accession numbers, gene symbols and names were retrieved from the Unigene Database (build#131).

**Table 7. Genes up-regulated in endometrial cysts throughout the menstrual cycle**

| Accession No. | Symbol | Gene name | OEX Assignment |
|---|---|---|---|
| M63262 | ALOX5AP | arachidonate 5-lipoxygenase-activating protein | 1 |
| X63629 | CDH3 | cadherin 3, type 1, P-cadherin (placental) | 6 |
| Z22970 | CD 163 | CD163 antigen | 186 |
| X04701 | C1R | complement component 1, r subcomponent | 68 |
| M20431 | | DC classII histocompatibility antigen alpha-chain | 187 |
| X00637 | HP | haptoglobin | 9 |
| K03431 | HPR | haptoglobin-related protein | 188 |
| V00497 | HBB | hemoglobin, beta | 189 |
| U01317 | HBD | hemoglobin, delta | 190 |
| AA583491 | HCA112 | hepatocellular carcinoma-associated antigen 112 | 2 |
| X00457 | | Human mRNA for SB classII histocompatibility antigen alpha-chain | 3 |
| M87789 | IGHG3 | immunoglobulin heavy constant gamma 3 (G3m marker) | **191** |
| M87790 | IGLλ | immunoglobulin lambda locus | 192 |
| K01171 | HLA-DRA | major histocompatibility complex, class II, DR alpha | 8 |
| X07819 | MMP7 | matrix metalloproteinase 7 (matrilysin, uterine) | 193 |
| U44403 | SLA | Src-like-adapter | 28 |
| Other Genes | | | |
| AI142828 | | Homo sapiens adlican mRNA, complete cds | 89 |
| K01505 | | ESTs | 5 |
| AI138545 | | ESTs | 194 |
| AI310156 | | ESTs, Weakly similar to A4P_HUMAN INTESTINAL MEMBRANE A4 PROTEIN [H. sapiens] | 15 |

Genes with normalized expression ratios (cyst/normal) of ≥5.0 in more than 50% of the 23 cases examined were selected. Accession Nos., gene symbols and names were retrieved from the Unigene Database (build #131).

**Table 8. Genes up-regulated in endometrial cysts from patients in the proliferative phase of the menstrual cycle**

| Accession No. | Symbol | Gene name | OEX Assignment |
|---|---|---|---|
| U07929 | ALDH6 | aldehyde dehydrogenase 6 | 195 |
| M86511 | CD 14 | CD 14 antigen | 11 |
| W67577 | CD74 | CD74 antigen (invariant polypeptide of major histocompatibility complex, class II antigen-associated) | 196 |
| U58514 | CHI3L2 | chitinase 3-like 2 | 197 |
| X02761 | FN1 | fibronectin 1 | 198 |
| AA854147 | HDAC7A | Histone deacetylase 7A | 199 |
| M83202 | LTF | lactotransferrin | 21 |
| X83006 | LCN2 | lipocalin 2 (oncogene 24p3) | 200 |
| M33906 | HLA-DQ A1 | major histocompatibility complex, class II, DQ alpha 1 | 201 |
| M15800 | MAL | mal, T-cell differentiation protein | 202 |
| AF034374 | | molybdenum cofactor biosynthesis protein A; molybdenum cofactor biosynthesis protein C | 20 |
| H48536 | PCAF | p300/CBP-associated factor | 203 |
| U02632 | KCNMA1 | potassium large conductance calcium-activated channel, subfamily M, alpha member 1 | 204 |
| AA972852 | RBP1 | retinol-binding protein 1, cellular | 205 |
| X00129 | RBP4 | retinol-binding protein 4, interstitial | 206 |
| L20688 | ARHGDI B | Rho GDP dissociation inhibitor (GDI) beta | 207 |
| AA778308 | RNASE1 | ribonuclease, RNase A family, (pancreatic) | 17 |
| X51441 | SAA1 | serum amyloid A1 | 208 |
| M77349 | TGFBI | transforming growth factor, beta-induced, 68kD | 4 |
| | | | |
| Other Genes | | | |
| AA455877 | | Homo sapiens cDNA FLJ11177 fis, clone PLACE1007402 | 209 |
| AA772709 | | Homo sapiens cDNA FLJ13522 fis, clone PLACE 1005884 | 210 |
| AA652120 | | Homo sapiens cDNA: FLJ21869 fis, clone HEP02442 | 211 |
| W87690 | | Homo sapiens cDNA: FLJ23173 fis, clone LNG10019 | 212 |
| AA938345 | | Homo sapiens mRNA; cDNA DKFZp564N1116 (from clone DKFZp564N1116) | 213 |
| AA577682 | | Homo sapiens mRNA; cDNA DKFZp586F1822 (from clone DKFZp586F1822) | 214 |
| N36090 | FLJ10895 | hypothetical protein FLJ10895 | 215 |
| AA164951 | | EST | 216 |
| H12942 | | ESTs | 47 |
| AA179600 | | ESTs | 217 |
| AI281932 | | ESTs | 218 |
| AI130715 | | ESTs | 219 |
| N66074 | | EST | 220 |
| AI038441 | | ESTs | 48 |
| W04197 | | ESTs | 221 |
| AA992745 | | ESTs | 222 |
| BE894625 | | EST | 53 |
| AA994249 | | ESTs | 49 |
| AA921763 | | ESTs | 52 |

Genes with normalized expression ratios (cyst/normal) of ≥5.0 in more than 50% of the nine cases examined were selected. Accession Nos., gene symbols and names were retrieved from the Unigene Database (build #131).

**Table 9. Genes up-regulated in endometrial cysts during the secretory phase of the menstrual cycle**

| Accession No. | Symbol | Gene name | |
|---|---|---|---|
| K03000 | ALDH1 | aldehyde dehydrogenase 1, soluble | 72 |
| 567310 | BF | B-factor, properdin | 62 |
| X56667 | CALB2 | calbindin 2, (29kD, calretinin) | 223 |
| T93566 | CPE | carboxypeptidase E | 224 |
| AA319695 | CEBPD | CCAAT/enhancer binding protein (C/EBP), delta | 60 |
| M14354 | F13A1 | coagulation factor XIII, A1 polypeptide | 225 |
| J04080 | C1S | complement component 1, s subcomponent | 64 |
| X04481 | C2 | complement component 2 | 74 |
| W45244 | C3 | complement component 3 | 58 |
| AA682870 | CCND2 | cyclin D2 | 75 |
| AF019413 | CYP21A2 | cytochrome P450, subfamily XXIA (steroid 21-hydroxylase, congenital adrenal hyperplasia), polypeptide 2 | 226 |
| D78014 | DPYSL3 | dihydropyrimidinase-like 3 | 78 |
| AA313118 | DUSP10 | dual specificity phosphatase 10 | 227 |
| AA573809 | ITLN | Intelectin | 228 |
| J00269 | KRT6A | keratin 6A | 229 |
| M81141 | HLA-DQ B1 | major histocompatibility complex, class II, DQ beta 1 | 10 |
| D28124 | NBL1 | neuroblastoma, suppression of tumorigenicity 1 | 59 |
| U20157 | PLA2G7 | phospholipase A2, group VII (platelet-activating factor acetylhydrolase, plasma) | 230 |
| D87258 | PRSS11 | protease, serine, 11 (IGF binding) | 86 |
| L35545 | PROCR | protein C receptor, endothelial (EPCR) | 231 |
| U70136 | PRG4 | proteoglycan 4, (megakaryocyte stimulating factor, articular superficial zone protein) | 13 |
| AA263000 | RNASE6 | ribonuclease, RNase A family, k6 | 232 |
| AF026692 | SFRP4 | secreted frizzled-related protein 4 | 233 |
| D29992 | TFPI2 | tissue factor pathway inhibitor 2 | 234 |
| X51630 | WT1 | Wilms tumor 1 | 235 |
| | | | |
| Other Genes | | | |
| AA429149 | C11ORF9 | chromosome 11open reading frame 9 | 236 |
| AI366242 | | ESTs | 237 |
| D85376 | | ESTs | 7 |
| M32093 | | ESTs | 14 |
| AA424195 | | ESTs | 238 |
| AI224952 | | ESTs | 239 |
| AA669034 | | Homo sapiens cDNA: FLJ23125 fis, clone LNG08217 | 240 |
| L02326 | | Homo sapiens clone Hu lambda7 lambda-like protein (IGLL2) gene, partial cds | 241 |
| D87465 | KIAA0275 | KIAA0275 gene product | 93 |
| AA528009 | KIAA1077 | KIAA107 protein | 242 |

Genes with normalized expression ratios (cyst/normal) of ≥5.0 in more than 50% of the 14 cases examined were selected. Accession Nos., gene symbols and names were retrieved from the Unigene Database (build #131).

### Diagnosing ovarian endometriosis

The present invention provides a method of diagnosing ovarian endometriosis. According to the present method, a subject suffering from or in the risk of developing ovarian endometriosis can be diagnosed. The subject diagnosed according to the present method is preferably a mammal. The mammal can be, for example, human, non-human primate, mouse, rat, dog, cat, horse or cow.

Ovarian endometriosis may be diagnosed by examining the expression of one or more OEX-associated genes in a subject-derived biological sample, (e.g., a subject-derived tissue sample) that contain or are suspected to contain an ovarian endometrial cell. Specifically, the method of diagnosing ovarian endometriosis of the present invention involves determining (measuring) the expression level of at least one and up to all the OEX associated genes listed in Tables 1-9. Using sequence information provided by the GenBank database entries for the known sequences, the ovarian endometriosis-associated genes are detected and measured using techniques well known to one of ordinary skill in the art. The expression of 1, 2, 3, 4, 5, 25, 35, 50, 100 or more of the sequences represented by OEX 1-242 may be determined and if desired, expression of these nucleotides can be determined along with other nucleotides whose expression level is known to be altered according to one of the herein described parameters or conditions, e.g., ovarian endometriosis or non-ovarian endometriosis.

According to the present method, the expression level of one or more of the ovarian endometriosis-associated gene in a biological sample derived from a subject is compared to a control level of the same gene. The biological sample may be any sample derived from the subject so long as the expression of the selected ovarian endometriosis-associated gene can be detected in a patient of ovarian endometriosis and includes individual cells and cell populations such as a test cells obtained from a bodily tissue or a bodily fluid (biological fluid such as blood, serum and urine). Preferably, the biological sample comprises a test cell population comprising an epithelial cell derived from a tissue that is known to be or suspected to be an endometrial cyst.

The control level of an ovarian endometriosis-associated gene in the diagnostic method of the present invention is the expression level of the same gene in a reference population. Cells in a reference cell population are derived from the same type of sample as that to the biological sample examined in the method. The reference cell population includes one or more cells for which the parameter to be compared is known, *i.e.,* endometriotic or non-endometriotic cell. Alternatively, a control cell population may be derived from a database of molecular information derived from cells for which the assay parameter or condition is known.

According to the present method of diagnosing ovarian endometriosis, the expression level of OEX nucleotide(s) in a subject-derived biological sample may be compared to multiple control levels of the OEX nucleotide(s). The control levels may be derived from biological samples with different known parameters (*i.e.*, endometriotic or non-endometriotic). Thus, the expression level of OEX nucleotide(s) in a subject-derived biological sample may be compared to the control level corresponding to ovarian endometrial cells (ovarian endometriosis control level), and then to the control corresponding to non-ovarian endometrial cells (normal cells) (normal control level). Thus, the control level may be a single expression pattern derived from a single reference population or may be a plurality of expression patterns. For example, the control level can be a database of expression patterns from previously tested cells.

Whether a pattern of gene expression levels in a subject-derived biological sample compared to that in the control level indicates ovarian endometriosis or a predisposition thereto depends on the kind of the sample used for determining the control level. For example, if the control level is detected in a sample of reference cell population composed of non-endometriotic cells, a similar gene expression level in the subject-derived biological sample indicates the subject to be non-endometriotic. Herein, such control levels are referred to as "normal control level". A normal control level indicates a level of gene expression detected in a normal, healthy individual or in a population of individuals known not to be suffering from ovarian endometriosis. A normal healthy individual is one with no clinical symptoms of ovarian endometriosis. Conversely, if the control level is detected in a sample of reference cell population made up of endometriotic cells, a similar gene expression profile between the subject-derived biological sample and the reference cell population indicates that the biological sample includes endometriotic cells. Herein, such control levels are referred to as "ovarian endometriosis control level". The phrase "ovarian endometriosis control level" refers to the expression profile of the endometriosis-associated genes found in a population suffering from endometriosis.

The expression level of an ovarian endometriosis-associated gene in a subject-derived biological sample can be considered as being altered if the expression level differs from the control level by more than 1.0, 1.5, 2.0, 5.0, 10.0 or more folds. Alternatively, an expression level of a gene in a subject-derived biological sample increased or decrease by 10%, 25%, 50% or more compared to the control level indicates alteration of the gene expression in the subject sample.

An increase in the level of OEX-1-97 and 186-242 detected in a subject-derived biological sample compared to a normal control level indicates that the subject suffers from or is at risk of developing ovarian endometriosis. In contrast, a decrease in the level of OEX 98-185 detected in a subject-derived biological sample compared to a normal control level indicates that the subject suffers from or is at risk of developing ovarian endometriosis.

A decrease or similarity in the expression level of OEX 1-97 and 186-242 detected in a subject-derived biological sample compared to an ovarian endometriosis control level indicates that the subject suffers from or is at risk of developing ovarian endometriosis. In contrast, an increase or similarity in the level of OEX 98-185 detected in a subject-derived biological sample compared to a normal control level indicates that said subject suffers from or is at risk of developing ovarian endometriosis.

Alteration in the expression of one or more of the ovarian endometriosis-associated genes in a subject-derived biological sample compared to the normal control level indicates that the subject suffers from or is at risk of developing ovarian endometriosis. To obtain a more reliable diagnosis result, it is preferable to examine the expression level of multiple OEX nucleotides. If the expression level of 1%, 5%, 25%, 50%, 60%, 80%, 90% or more of the OEX 1-242 is altered in a subject-derived biological sample, the probability that the subject suffers from or is at risk of developing ovarian endometriosis becomes quite high.

If desired, comparison of the expression level of a gene in a subject-derived biological sample to the control level can be conducted with respect to a control nucleic acid whose expression is independent of the parameter or condition being measured. A "control nucleic acid" is one whose expression is known not to differ between the endometriotic or non-endometriotic state of the cell. Expression levels of the control nucleic acid in the test and reference nucleic acid can be used to normalize signal levels in the compared populations. A control nucleic acid is also called "housekeeping gene" and genes such as β-actin, glyceraldehyde 3-phosphate dehydrogenase or ribosomal protein P1 may be used in the present invention.

Expression of the genes disclosed herein may be determined at the RNA level using any method known in the art. For example, sequences within the sequence database entries corresponding to OEX nucleic acid sequences can be used to construct probes for detecting OEX RNAs by, *e.g.,* Northern blot hybridization analyses. A probe preferably includes at least 10, 20, 50, 100, 200 or more continuous nucleotides of a reference sequence (*i.e*., the nucleotide sequence of OEX nucleic acids (Tables 1-9)). Alternatively, the expression level is measured using reverse-transcription-based PCR (RT-PCR) assays, e.g., using primers specific for the OEX nucleic acid sequences.

According to the method, the expression level can also be determined at the protein level by measuring the levels or the biological activity of the expressed polypeptides encoded by the genes described herein. Such methods are well known in the art and include immunoassays based on antibodies against polypeptides encoded by the genes. The biological activities of respective polypeptides encoded by the ovarian endometriosis-associated genes are also well known in the art, and one skilled in the art can adopt appropriate conventional methods for measuring the biological activities of the polypeptides depending on the kind of polypeptide to be measured.

When alterations in gene expression are associated with gene amplification or deletion, the gene (DNA sequence) in a subject-derived biological sample may be compared to that of a reference cell population to determine whether the subject suffers from or is at risk of developing ovarian endometriosis.

### Assessing efficacy of treatment of ovarian endometriosis in a subject

The differentially expressed OEX nucleotides identified herein also allow for the course of treatment of ovarian endometriosis to be monitored. According to the method, a biological sample, such as a test cell population, is obtained from a subject undergoing treatment for ovarian endometriosis. The method for assessment can be conducted according to the method of diagnosing ovarian endometriosis of the present invention described above.

If desired, biological samples are obtained from the subject at various time points before, during or after the treatment. The expression level of one or more of the OEX associated genes, in the biological sample is then determined and compared to a control level derived, for example, from a reference cell population which includes cells whose state of ovarian endometriosis (*i.e*., endometriotic or non-endometriotic) is known. The control level is determined in a biological sample that has not been exposed to the treatment.

If the control level is derived from a biological sample which contains no ovarian endometrial cells, a similarity between the expression level in the subject-derived biological sample and the control level indicates that the treatment is efficacious. A difference between the expression level of the OEX nucleotides in the subject-derived biological sample and the control level indicates a less favorable clinical outcome or prognosis.

The term "efficacious" refers that the treatment leads to a reduction in the expression of a pathologically up-regulated gene (OEX 1-97 and OEX 186-242), increase in the expression of a pathologically down-regulated gene (OEX 98-185) or a decrease in size, prevalence or proliferating potential of endometrial cysts in a subject. When a treatment is applied prophylactically, "efficacious" indicates that the treatment retards or prevents formation of ovarian endometrial cysts. The assessment of endometrial cysts can be made using standard clinical protocols.

The efficaciousness of a treatment is determined in association with any known method for diagnosing or treating ovarian endometriosis. Ovarian endometriosis is diagnosed for example, by identifying symptomatic anomalies, e.g., progressive dysmenorrhea, dyspareunia, chronic pelvic pain and infertility, along with surgical identification of endometrial glands and stroma outside the uterus.

### Assessing the prognosis of a subject with ovarian endometriosis

The present invention further provides a method of assessing the prognosis of a patient with ovarian endometriosis by comparing the expression level of one or more OEX nucleotides in a patient-derived biological sample, such as test cell population, to a control level. Alternatively, the expression level of one or more OEX nucleotides in a biological sample derived from patients may be measured over a spectrum of disease stages to assess the prognosis of the patient. The method for assessment can be conducted according to the method of diagnosing ovarian endometriosis of the present invention described above.

A decrease in the expression level of one or more of OEX 98-185 compared to a normal control level or an increase of the expression level of one or more of OEX 1-97 and 186-242 compared to a normal control indicates less favorable prognosis. An increase in the expression level of one or more of OEX 98-185 indicates a more favorable prognosis, and a decrease in the expression level of OEX 1-97 and 186-242 indicates a more favorable prognosis for the patient.

### Ovarian endometriosis reference expression profile

An ovarian endometriosis reference expression profile is provided by the present invention. Such expression profiles of the present invention comprise a pattern of gene expression of two or more OEX nucleotides (OEX 1-242) in a cell of endometrial cysts or a normal healthy endometrial cell (non-endometriotic cell). Furthermore, the present expression profile may comprise a pattern of gene expression of two or more genes of OEX 1-97 and OEX 186-242 or OEX 98-185. The expression profile can be used in diagnosing ovarian endometriosis or a predisposition to developing ovarian endometriosis in a subject, monitoring the course of treatment of ovarian endometriosis and assessing prognosis of a subject with ovarian endometriosis.

### Screening compounds that alter ovarian endometriosis-associated gene expression or the biological activity of a polypeptide encoded by the ovarian endometriosis-associated gene

The invention further provides methods of screening a compound that alters, *i.e.,* inhibits or enhances the expression of a marker gene, ovarian endometriosis-associated gene (OEX 1-242), by (1) contacting a test compound with a test cell expressing an ovarian endometriosis-associated gene or a cell into which a vector comprising a reporter gene linked downstream of a transcriptional regulatory region of an ovarian endometriosis-associated gene has been introduced; (2) determining the expression level of the ovarian endometriosis-associated gene; and (3) selecting the compound that alters the expression level compared to that in the absence of the test compound. The method is based on screening a compound to determine if it converts an expression profile of OEX 1-242 characteristic of an ovarian endometriosis state to a pattern indicative of a non-ovarian endometriosis state.

A decrease in the expression level of an ovarian endometriosis-associated gene or a reporter gene linked downstream of a transcriptional regulatory region of an ovarian endometriosis-associated gene compared to the expression level detected in the absence of a test compound indicates that the test compound is an inhibitor. Alternatively, enhanced expression level of the genes compared to the expression level detected in the absence of a test compound indicates the test compound to function as an enhancer. The expression level detected in the absence of a test compound may be a normal control level or ovarian endometriosis control level.

If a gene up-regulated in the endothelial cells of endometrial cysts of patients with ovarian endometriosis (e.g., any one selected from OEX 1-97 and OEX 186-242) or the transcriptional regulatory region thereof is used in the screening method, a compound that inhibits the expression of the gene is expected to inhibit endometriosis.

Alternatively, if a gene down-regulated in the endothelial cells of endometrial cysts of patients with ovarian endometriosis (e.g., any one selected from OEX 98-185) or the transcriptional regulatory region thereof is used in the screening method, a compound that enhances the expression of the gene is expected to inhibit endometriosis.

In the method, a cell may be exposed to a test compound or a combination of test compounds (sequentially or consequentially). Compounds selected by the screening of the present invention serve as candidate compounds for treating or preventing ovarian endometriosis. Compounds effective in stimulating expression of underexpressed (down-regulated) marker genes or in suppressing expression of overexpressed (up-regulated) marker genes are deemed to lead to a clinical benefit. Therefore, such compounds are further tested for the ability to prevent endometrial cyst growth in endometrial glands and/or stroma of animals or test subjects. Moreover, compounds in which a part of the structure is converted by addition, deletion, substitution and/or insertion are also included in the compounds obtainable by the screening of the present invention.

The test cell used in the screening may be any cell so long as it expresses the ovarian endometriosis-associated gene. Furthermore, the test cell may be a test cell population consisting of multiple cells. For example, the test cell or test cell population contains an epithelial cell, such as those derived from an endometrial cyst. Moreover, the test cell or test cell population may be an immortalized cell or cell line derived from an endometrial cyst cell.

A transcriptional regulatory region of an ovarian endometriosis-associated gene can be obtained from genomic libraries using probes comprising the 5' region of the OEX nucleotides (OEX 1-242). Any reporter gene may be used in the screening so long as its expression can be detected in the screening. Examples of reporter genes include the β-gal gene, CAT gene and luciferase gene. Detection of the expression of the reporter gene can be conducted based on conventional methods in accordance with the type of the reporter gene. Although there is no restriction on the cell into which the vector is introduced, preferable examples include epithelial cell.

The present invention further provides a method of screening for a compound that alters the activity of an ovarian endometriosis-associated gene. An embodiment of this screening method comprises the steps of: (a) contacting a test compound with a polypeptide encoded by an ovarian endometriosis-associated gene; (b) detecting the binding activity between the polypeptide and the test compound; and (c) selecting the compound that binds to the polypeptide.

In another embodiment of the method for screening a compound that alters the activity of an ovarian endometriosis-associated gene, the method utilizes the biological activity of an OEX polypeptide as an index. The screening method includes the steps of.
(a) contacting a test compound with a polypeptide encoded by an ovarian endometriosis-associated gene; (b) detecting the biological activity of the polypeptide; and
(c) selecting the compound that alters the biological activity of the polypeptide in comparison with the biological activity detected in the absence of the test compound.

The OEX polypeptides used for the present screening are selected from:
(1) a polypeptide comprising the amino acid sequence encoded by a polynucleotide selected from the group consisting of OEX 1-242;
(2) a polypeptide that comprises the amino acid sequence encoded by a polynucleotide selected from the group consisting of OEX 1-242, in which one or more amino acids are substituted, deleted and/or added and that has a biological activity equivalent to a protein consisting of the amino acid sequence encoded by the polynucleotide; and
(3) a polypeptide encoded by a polynucleotide that hybridizes under stringent conditions to a polynucleotide selected from the group consisting of OEX 1-242, wherein the polypeptide has a biological activity equivalent to a polypeptide consisting of the amino acid sequence encoded by the polynucleotide selected from the group consisting of OEX 1-242.

In the present invention, the phrase "biological activity" refers to activities such as occurrence, growth or proliferation of endometrial cyst cell. Whether an objective polypeptide has the biological activity or not can be judged by introducing the polypeptide or a DNA encoding the polypeptide into a cell, and detecting growth or proliferation of the cells, inrease in colony forming activity, etc.

Methods for preparing polypeptides having the biological activity of a given protein are well known in the art and include methods introducing mutations into the protein. For example, one can prepare polypeptides having the biological activity of the OEX protein by introducing an appropriate mutation in the amino acid sequence of either of these proteins by site-directed mutagenesis (Hashimoto-Gotoh et al. (1995) Gene 152: 271-5; Zoller and Smith (1983) Methods Enzymol. 100: 468-500; Kramer et al. (1984) Nucleic Acids Res. 12: 9441-56; Kramer and Fritz (1987) Methods Enzymol. 154: 350-67; Kunkel (1985) Proc. Natl. Acad. Sci. USA 82: 488-92; Kunkel (1988) Methods Enzymol. 85: 2763-6). Amino acid mutations can occur in nature too. The OEX polypeptides include those having the amino acid sequences of the human OEX proteins in which one or more amino acids are mutated, provided the resulting mutated polypeptides have the biological activity of the OEX proteins. The number of amino acids to be mutated in such a mutant is generally 10 amino acids or less, preferably 6 amino acids or less and more preferably 3 amino acids or less.

An example of a polypeptide to which one ore more amino acid residues are added include fusion proteins containing the OEX protein. Fusion proteins can be made by techniques well known to those skilled in the art, such as linking DNA encoding the OEX protein with DNA encoding other peptides or proteins, so that the frames match, inserting the fused DNA into an expression vector and expressing it in a host. There is no restriction as to the peptides or proteins fused to the OEX protein and include FLAG (Hopp et al. (1988) Biotechnology 6: 1204-10), 6xHis, 10xHis, Influenza agglutinin, human c-myc fragment, VSP-GP fragment, p18HIV fragment, T7-tag, HSV-tag, E-tag, SV40T antigen fragment, lck tag, α-tubulin fragment, B-tag, Protein C fragment, glutathione-S-transferase, immunoglobulin constant region, β-galactosidase, maltose binding protein, green fluorescence protein, etc. Vectors which can express a fusion protein with such peptides or proteins by the use of its multiple cloning sites are commercially available and can be used for obtaining fusion proteins to be used in the present screening.

An alternative method known in the art to isolate polypeptides having the biological activity of any of the OEX proteins is, for example, the method using hybridization technique (Sambrook et al. (1989) Molecular Cloning 2nd ed. 9.47-9.58, Cold Spring Harbor Lab. Press). One skilled in the art can readily isolate a DNA having high homology with a whole or part of the DNA sequence encoding an OEX protein, and isolate polypeptides having the biological activity of the OEX protein from the isolated DNA. The OEX polypeptides include those that are encoded by DNA that hybridize with a whole or part of a gene selected from OEX 1-242 and have the biological activity of the OEX protein. These polypeptides include mammal homologues corresponding to the protein derived from human (for example, a polypeptide encoded by a monkey, rat, rabbit and bovine gene). In isolating a cDNA highly homologous to a gene selected from OEX 1-242 from animals, it is particularly preferable to use tissues from colorectal cancers with metastasis.

In place of hybridization, a gene amplification method, for example, the PCR method, can be utilized to isolate a DNA encoding a polypeptide having the biological activity of the OEX protein, using a primer synthesized based on the sequence information of the protein encoding DNA (OEX 1-242).

An OEX polypeptide used in the method of the present invention may have variations in amino acid sequence, molecular weight, isoelectric point, the presence or absence of sugar chains or form, depending on the cell or host used to produce it or the purification method utilized. Nevertheless, so long as it has a biological activity equivalent to that of the OEX protein, it may be used in the method of the present invention and such methods utilizing polypeptides with a biological activity equivalent to the OEX protein are within the scope of the present invention.

The OEX polypeptides used in the present invention can be prepared as recombinant proteins or natural proteins, by methods well known to those skilled in the art. A recombinant protein can be prepared by inserting a DNA, which encodes the OEX polypeptide, into an appropriate expression vector, introducing the vector into an appropriate host cell, obtaining the extract, and purifying the polypeptide. Alternatively, a natural protein can be isolated by methods known to a person skilled in the art, for example, by contacting the affinity column, in which antibodies binding to the OEX protein described below are bound, with the extract of tissues or cells expressing the OEX polypeptide. The antibodies can be polyclonal antibodies or monoclonal antibodies.

The OEX polypeptide to be contacted with a test compound can be, for example, a purified polypeptide, a soluble protein, a form bound to a carrier or a fusion protein fused with other polypeptides. Examples of supports that may be used for binding proteins include insoluble polysaccharides, such as agarose, cellulose and dextran; and synthetic resins, such as polyacrylamide, polystyrene and silicon; preferably commercial available beads and plates (multi-well plates, biosensor chip, etc.) prepared from the above materials may be used. When using beads, they bay be filled into a column.

The binding of a protein to a support may be conducted according to routine methods, such as chemical bonding and physical adsorption. Alternatively, a protein may be bound to a support via antibodies that specifically recognizing the protein. Moreover, binding of a protein to a support can be also conducted by means of avidin and biotin binding.

As a method of screening for proteins, for example, that bind to the OEX polypeptide using any of the OEX polypeptides described above, many methods well known by a person skilled in the art can be used. Such a screening can be conducted by, for example, immunoprecipitation method, specifically, in the following manner.

In immunoprecipitation, an immune complex is formed by adding an antibody to cell lysate prepared using an appropriate detergent. The antibody used in the immunoprecipitation for the screening recognizes any of the proteins endcoded by OEX 1-242. Alternatively, when an OEX protein fused with a recognition site (epitope) is used in the screening, antibodies against the epitope may be used for the immunoprecipitaion. The immune complex consists of the OEX protein, a polypeptide comprising the binding ability with the OEX protein, and an antibody.

An immune complex can be precipitated, for example by Protein A sepharose or Protein G sepharose when the antibody is a mouse IgG antibody. If the OEX polypeptide is prepared as a fusion protein with an epitope, such as GST, an immune complex can be formed in the same manner as in the use of the antibody against the OEX polypeptide, using a substance specifically binding to these epitopes, such as glutathione-Sepharose 4B.

Immunoprecipitation can be performed by following or according to, for example, the methods in the literature (Harlow and Lane (1988) Antibodies, 511-52, Cold Spring Harbor Laboratory publications, New York).

SDS-PAGE is commonly used for analysis of immunoprecipitated proteins and the bound protein can be analyzed by the molecular weight of the protein using gels with an appropriate concentration. Since the protein bound to the OEX polypeptide may be difficult to detect by a common staining method, such as Coomassie staining or silver staining, the detection sensitivity for the protein can be improved by culturing cells in culture medium containing radioactive isotope, ³⁵S-methionine or ³⁵S-cystein, labeling proteins in the cells, and detecting the proteins. The target protein can be purified directly from the SDS-polyacrylamide gel and its sequence can be determined, when the molecular weight of a protein has been revealed.

As a method for screening proteins binding to the OEX polypeptide using the polypeptide, for example, West-Western blotting analysis (Skolnik et al. (1991) Cell 65: 83-90) can be used. Specifically, a protein binding to the OEX polypeptide can be obtained by preparing a cDNA library from cells, tissues, organs or cultured cells expected to express a protein binding to the OEX polypeptide using a phage vector (*e.g.*, ZAP), expressing the protein on LB-agarose, fixing the protein expressed on a filter, reacting the purified and labeled OEX polypeptide with the above filter, and detecting the plaques expressing proteins bound to the OEX polypeptide according to the label. The OEX polypeptide may be labeled by utilizing the binding between biotin and avidin, or by utilizing an antibody that specifically binds to the OEX polypeptide, or a peptide or polypeptide (for example, GST) that is fused to the OEX polypeptide. Methods using labeling substances such as radioisotope (³H, ¹⁴C, ³²P, ³³P, ³⁵S, ¹²⁵I, ¹³¹I, etc.), enzymes (alkaline phosphatase, horseradish peroxidase, β-galactosidase, β-glucosidase, etc.), fluorescent substances (fluorescein isothiosyanete (FITC), rhodamine, etc.) and biotin/avidin, may be used for the labeling in the present method. When the OEX protein is labeled with radioisotope, the detection or measurement can be carried out by liquid scintillation. Alternatively, OEX proteins labeled with enzymes can be detected or measured by adding a substrate of the enzyme to detect the enzymatic change of the substrate, such as generation of color, with absorptiometer. Further, in case where a fluorescent substance is used as the label, the bound protein may be detected or measured using fluorophotometer.

Alternatively, in another embodiment of the screening method of the present invention, a two-hybrid system utilizing cells may be used ("MATCHMAKER Two-Hybrid system", "Mammalian MATCHMAKER Two-Hybrid Assay Kit", "MATCHMAKER one-Hybrid system" (Clontech); "HybriZAP Two-Hybrid Vector System" (Stratagene); the references "Dalton and Treisman (1992) Cell 68: 597-612", "Fields and Sternglanz (1994) Trends Genet 10: 286-92").

In the two-hybrid system, the OEX polypeptide is fused to the SRF-binding region or GAL4-binding region and expressed in yeast cells. A cDNA library is prepared from cells expected to express a protein binding to the OEX polypeptide, such that the library, when expressed, is fused to the VP16 or GAL4 transcriptional activation region. The cDNA library is then introduced into the above yeast cells and the cDNA derived from the library is isolated from the positive clones detected (when a protein binding to the OEX polypeptide is expressed in yeast cells, the binding of the two activates a reporter gene, making positive clones detectable). A protein encoded by the cDNA can be prepared by introducing the cDNA isolated above to *E. coli* and expressing the protein.

As a reporter gene, for example, Ade2 gene, lacZ gene, CAT gene, luciferase gene and such can be used besides HIS3 gene.

A compound binding to the OEX polypeptide can also be screened using affinity chromatography. For example, the OEX polypeptide may be immobilized on a carrier of an affinity column, and a test compound, containing a protein capable of binding to the ODX polypeptide, is applied to the column. A test compound herein may be, for example, cell extracts, cell lysates, etc. After loading the test compound, the column is washed, and compounds bound to the OEX polypeptide can be prepared.

When the test compound is a protein, the amino acid sequence of the obtained protein is analyzed, an oligo DNA is synthesized based on the sequence, and cDNA libraries are screened using the oligo DNA as a probe to obtain a DNA encoding the protein.

A biosensor using the surface plasmon resonance phenomenon may be used as a mean for detecting or quantifying the bound compound in the present invention. When such a biosensor is used, the interaction between the OEX polypeptide and a test compound can be observed real-time as a surface plasmon resonance signal, using only a minute amount of polypeptide and without labeling (for example, BIAcore, Pharmacia). Therefore, it is possible to evaluate the binding between the OEX polypeptide and a test compound using a biosensor such as BIAcore.

The methods of screening for molecules that bind when the immobilized OEX polypeptide is exposed to synthetic chemical compounds, or natural substance banks, or a random phage peptide display library, or the methods of screening using high-throughput based on combinatorial chemistry techniques (Wrighton et al. (1996) Science 273: 458-64; Verdine (1996) Nature 384: 11-13; Hogan (1996) Nature 384: 17-9) to isolate not only proteins but chemical compounds that bind to the OEX protein (including agonist and antagonist) are well known to those skilled in the art.

Alternatively, when the biological activity of the OEX polypeptide is detected in the screening of the present invention, a compound isolated by this screening is a candidate for agonists or antagonists of the OEX polypeptide. The term "agonist" refers to molecules that activate the function of the OEX polypeptide by binding thereto. Likewise, the term "antagonist" refers to molecules that inhibit the function of the OEX polypeptide by binding thereto. Moreover, a compound isolated by this screening is a candidate for compounds which inhibit the *in vivo* interaction of the OEX polypeptide with molecules (including DNAs and proteins).

When the biological activity to be detected in the present method is cell proliferation, it can be detected, for example, by preparing cells which express the OEX polypeptide, culturing the cells in the presence of a test compound, and determining the speed of cell proliferation, measuring the cell cycle and such, as well as by measuring the colony forming activity.

A decrease in the binding activity or biological activity of one or more polypeptides encoded by OEX 1-97 and OEX 186-242 compared to a normal control level of the gene detected by the screening method indicates that the test compound is an inhibitor of the ovarian endometriosis-associated gene and is expected to reduce the symptom of endometriosis. Alternatively, an increase of the binding activity with or the biological activity of one or more polypeptides encoded by OEX 98-185 compared to a normal control level of the gene detected by the screening method indicates that the test compound is an enhancer of the ovarian endometriosis-associated gene and is expected to reduce the symptom of endometriosis. A compound isolated by the above screenings is a candidate for drugs which can be applied for the treatment or prevention of ovarian, endometriosis. Moreover, compound in which a part of the structure of the compound that alters the activity of the OEX protein is converted by addition, deletion and/or replacement are also included in the compounds obtainable by the screening method of the present invention.

Any test compound, for example, cell extracts, cell culture supernatant, products of fermenting microorganism, extracts from marine organism, plant extracts, purified or crude proteins, peptides, non-peptide compounds, synthetic micromolecular compounds and natural compounds can be used in the screening methods of the present invention. The test compound of the present invention can be also obtained using any of the numerous approaches in combinatorial library methods known in the art, including (1) biological libraries, (2) spatially addressable parallel solid phase or solution phase libraries, (3) synthetic library methods requiring deconvolution, (4) the "one-bead one-compound" library method and (5) synthetic library methods using affinity chromatography selection. The biological library methods using affinity chromatography selection is limited to peptide libraries, while the other four approaches are applicable to peptide, non-peptide oligomer or small molecule libraries of compounds (Lam (1997) Anticancer Drug Des. 12: 145). Examples of methods for the synthesis of molecular libraries can be found in the art (DeWitt et al. (1993) Proc. Natl. Acad. Sci. USA 90: 6909; Erb et al. (1994) Proc. Natl. Acad. Sci. USA 91: 11422; Zuckermann et al. (1994) J. Med. Chem. 37: 2678; Cho et al. (1993) Science 261: 1303; Carell et al. (1994) Angew. Chem. Int. Ed. Engl. 33: 2059; Carell et al. (1994) Angew. Chem. Int. Ed. Engl. 33: 2061; Gallop et al. (1994) J. Med. Chem. 37: 1233). Libraries of compounds may be presented in solution (see Houghten (1992) Bio/Techniques 13: 412) or on beads (Lam (1991) Nature 354: 82), chips (Fodor (1993) Nature 364: 555), bacteria (US Pat. No. 5,223,409), spores (US Pat. No. 5,571,698;5,403,484, and 5,223,409), plasmids (Cull et al. (1992) Proc. Natl. Acad. Sci. USA 89: 1865) or phage (Scott and Smith (1990) Science 249: 386; Delvin (1990) Science 249: 404; Cwirla et al. (1990) Proc. Natl. Acad. Sci. USA 87: 6378; Felici (1991) J. Mol. Biol. 222: 301; US Pat. Application 2002103360)

### Selecting a therapeutic agent for treating ovarian endometriosis appropriate for a particular individual

Differences in the genetic makeup of individuals can result in differences in their relative abilities to metabolize various drugs. A compound that is metabolized in a subject to act as an anti-ovarian endometriosis agent can manifest itself by inducing a change in gene expression pattern in the subject's cells from that characteristic of an ovarian endometriosal state to a gene expression pattern characteristic of a non-ovarian endometriosal state. Accordingly, the differentially expressed OEX associated genes disclosed herein allow for selection of a putative therapeutic or prophylactic anti-ovarian endometriosis agent specifically adequate for a subject by testing candidate compounds in a test cell population from the selected subject.

To identify an anti-ovarian endometriosis agent, that is appropriate for a specific subject, a test cell or test cell population derived from the subject is exposed to a candidate therapeutic agent and the expression of one or more of OEX 1-242 genes is determined.

The test cell is or the test cell population contains an ovarian endometrial cell expressing an ovarian endometriosis associated gene. Preferably, the test cell is or the test cell population contains an epithelial cell. For example, a test cell or test cell population is incubated in the presence of a candidate agent and the pattern of gene expression in the test cell or cell population is measured and compared to one or more reference profiles (an ovarian endometriosis reference expression profile or an non-ovarian endometriosis reference expression profile).

A decrease in the expression of one or more of OEX 1-97 and 186-242 or an increase in the expression of one or more of OEX 98-185 in a test cell or test cell population relative to that in a reference cell population containing ovarian endometriosis is indicative that the agent is therapeutic.

The test agent can be any compound or composition. For example, the test agent is an immunomodulatory agent.

### Kit

The invention also provides a kit comprising an OEX-detection reagent, e.g., a nucleic acid that specifically binds to or identifies one or more of OEX nucleic acids. Such nucleic acid specifically binding to or identifying one or more of OEX nucleic acids are exemplified by oligonucleotide sequences that are complementary to a portion of an OEX nucleic acid or antibodies which bind to polypeptides encoded by an OEX nucleic acid. The reagents are packaged together in the form of a kit. The reagents such as a nucleic acid or antibody (either bound to a solid matrix or packaged separately with reagents for binding them to the matrix), a control reagent (positive and/or negative) and/or a means of detection of the nucleic acid or antibody are preferably packaged in separate containers. Instructions (written, tape, VCR, CD-ROM, etc.) for carrying out the assay are included in the kit. The assay format of the kit may be Northern hybridization or a sandwich ELISA known in the art.

For example, an OEX detection reagent is immobilized on a solid matrix such as a porous strip to form at least one OEX detection site. The measurement or detection region of the porous strip may include a plurality of detection sites, each detection site containing an OEX-detection reagent. A test strip may also contain sites for negative and/or positive controls. Alternatively, control sites are located on a separate strip from the test strip. Optionally, the different detection sites may contain different amounts of immobilized nucleic acids, *i.e*., a higher amount in the first detection site and lesser amounts in subsequent sites. Upon the addition of a test biological sample, the number of sites displaying a detectable signal provides a quantitative indication of the amount of OEX present in the sample. The detection sites may be configured in any suitably detectable shape and are typically in the shape of a bar or dot spanning the width of a teststrip.

Alternatively, the kit contains a nucleic acid substrate array comprising one or more ovarian endometriosis-associated genes. The nucleic acids on the array specifically identify one or more nucleic acid sequences represented by OEX 1-242. The expression level of 2, 3, 4, 5, 6, 7, 8, 9, 10, 15, 20, 25, 40 or 50 or more of the sequences represented by OEX 1-242 are identified by virtue if the level of binding to an array test strip or chip. The substrate array can be on a solid substrate, *e.g.,* a "chip" as described in US Patent No.5,744,305.

### Arrays and pluralities

The invention also includes a nucleic acid substrate array comprising one or more ovarian endometriosis-associated genes. The nucleic acids on the array specifically corresponds to one or more nucleic acid sequences represented by OEX 1-242. The expression level of 2, 3, 4, 5, 6, 7, 8, 9, 10, 15, 20, 25, 40 or 50 or more of the OEX nucleic acids represented by OEX 1-242 are identified by detecting the binding of nucleotides to the array.

The invention also includes an isolated plurality of nucleic acids (*i.e*., a mixture if two or more OEX nucleic acids). The nucleic acids are in a liquid phase or a solid phase, e.g., immobilized on a solid support such as a nitrocellulose membrane. The plurality includes one or more of the nucleic acids represented by OEX 1-242. According to a further embodiment of the present invention, the plurality includes 2, 3, 4, 5, 6, 7, 8, 9, 10, 15, 20, 25, 40 or 50 or more of the nucleic acids represented by OEX 1-242.

### Method of treating or preventing ovarian endometriosis

The invention provides a method for treating or preventing ovarian endometriosis in a subject. Therapeutic compounds are administered prophylactically or therapeutically to subject suffering from or at risk of (or susceptible to) developing endometriosis. Such subjects are identified using standard clinical methods or by detecting an aberrant expression level or activity of OEX 1-242. Prophylactic administration occurs prior to the manifestation of overt clinical symptoms of disease, such that a disease or disorder is prevented or, alternatively, delayed in its progression.

The therapeutic method includes increasing the expression or function, or both of one or more gene products of genes whose expression is decreased ("under-expressed genes") in an ovarian endometrial cell relative to normal cells of the same tissue type from which the ovarian endometrial cells are derived. In these methods, the subject is treated with an effective amount of a compound, which increases the amount of one of more of the under-expressed genes (OEX 97-185) in the subject. Administration can be systemic or local. Therapeutic compounds include polypeptide products of the under-expressed gene, or a biologically active fragment thereof, a nucleic acid encoding an under-expressed gene downstream of expression control elements permitting expression of the gene in the ovarian endometrial cells, compounds that increase the expression level of such gene endogenously existing in the ovarian endometrial cells (i. e., compounds that up-regulate the expression of the under-expressed gene(s)). Administration of such therapeutic compounds counter the effects of aberrantly-under expressed gene(s) in the subjects' ovarian cells and are expected to improve the clinical condition of the subject. Such compounds can be obtained by the screening method of the present invention described above.

The method also includes decreasing the expression or function, or both, of one or more gene products of genes whose expression is aberrantly increased ("over-expressed gene") in ovarian endometrial cells. In these methods, the subject is treated with an effective amount of a compound, which decreases the amount of one of more of the over-expressed genes (OEX 1-96 and 186-242) in the subject. Administration can be systemic or local. Therapeutic compounds include compounds that decrease the expression level of such gene endogenously existing in the ovarian endometrial cells *(i.e.,* compounds that down-regulate the expression of the over-expressed gene(s)). Administration of such therapeutic compounds counter the effects of aberrantly-over expressed gene(s) in the subjects ovarian cells and are expected to improve the clinical condition of the subject. Such compounds can be obtained by the screening method of the present invention described above.

The expression of over-expressed genes may be also inhibited in any of several ways known in the art including administering to the subject a nucleic acid that inhibits or antagonizes the expression of the over-expressed gene(s). Antisense oligonucleotides, siRNA or ribozymes which disrupts expression of the over-expressed gene(s) can be used for inhibiting the expression of over-expressed genes.

As noted above, antisense-oligonucleotides corresponding to any of the nucleotide sequence of OEX 1-97 or OEX 186-242 can be used to reduce the expression level of the OEX 1-97 or OEX 186-242. Antisense-oligonucleotides corresponding to OEX 1-97 or OEX 186-242 that are up-regulated in ovarian endometriosis are useful for the treatment or prevention of ovarian endometriosis. Specifically, the antisense-oligonucleotides of the present invention may act by binding to any of the polypeptides encoded by the OEX 1-97 or OEX 186-242, or mRNAs corresponding thereto, thereby inhibiting the transcription or translation of the genes, promoting the degradation of the mRNAs, and/or inhibiting the expression of proteins encoded by the OEX nucleotides, and finally inhibiting the function of the proteins. The term "antisense-oligonucleotides" as used herein encompasses both nucleotides that are entirely complementary to the target sequence and those having a mismatch of one or more nucleotides, so long as the antisense-oligonucleotides can specifically hybridize to the target sequence. For example, the antisense-oligonucleotides of the present invention include polynucleotides that have a homology of at least 70% or higher, preferably at 80% or higher, more preferably 90% or higher, even more preferably 95% or higher over a span of at least 15 continuous nucleotides to any of the nucleotide sequence of OEX 1-97 and OEX 186-242. Algorithms known in the art can be used to determine the homology. Furthermore, derivatives or modified products of the antisense-oligonucleotides can also be used as antisense-oligonucleotides in the present invention. Examples of such modified products include lower alkyl phosphonate modifications such as methyl-phosphonate-type or ethyl-phosphonate-type, phosphorothioate modifications and phosphoroamidate modifications.

The antisense-oligonucleotides and derivatives thereof act on cells producing the proteins encoded by marker genes (OEX 1-97, OEX 186-242) by binding to the DNAs or mRNAs encoding the proteins, inhibiting their transcription or translation, promoting the degradation of the mRNAs and inhibiting the expression of the proteins, thereby resulting in the inhibition of the protein function.

An antisense-oligonucleotides and derivatives thereof can be made into an external preparation, such as a liniment or a poultice, by mixing with a suitable base material which is inactive against the derivative.

The antisense-oligonucleotides of the invention inhibit the expression of at least one OEX protein encoded by any one of OEX 1-97 and OEX 186-242, and thus is useful for suppressing the biological activity of the protein.

The nucleic acids that inhibit one or more gene products of overexpressed genes also include small interfering RNAs (siRNA) comprising a combination of a sense strand nucleic acid and an antisense strand nucleic acid of the nucleotide sequence encoding an over-expressed OEX protein, such as OEX 1-97 and 186-242. The term "siRNA" refers to a double stranded RNA molecule which prevents translation of a target mRNA. Standard techniques of introducing siRNA into the cell can be used in the treatment or prevention of the present invention, including those in which DNA is a template from which RNA is transcribed. The siRNA is constructed such that a single transcript has both the sense and complementary antisense sequences from the target gene, e.g., a hairpin.

The method is used to suppress gene expression of a cell with up-regulated expression of an OEX gene. Binding of the siRNA to the OEX gene transcript in the target cell results in a reduction of OEX protein production by the cell. The length of the oligonucleotide is at least 10 nucleotides and may be as long as the naturally occurring transcript. Preferably, the oligonucleotide is 19-25 nucleotides in length. Most preferably, the oligonucleotide is less than 75, 50 or 25 nucleotides in length.

The nucleotide sequence of siRNAs may be designed using a siRNA design computer program available from the Ambion website (http://www.ambion.com/techlib/misc/siRNA_finder.html). Nucleotide sequences for the siRNA are selected by the computer program based on the following protocol:

### Selection of siRNA Target Sites:

1. Beginning with the AUG start codon of transcript, scan downstream for AA dinucleotide sequences. Record the occurrence of each AA and the 3' adjacent 19 nucleotides as potential siRNA target sites. Tuschl, et al. recommend not to design siRNA against the 5' and 3' untranslated regions (UTRs) and regions near the start codon (within 75 bases) as these may be richer in regulatory protein binding sites, and thus the complex of endonuclease and siRNAs that were designed against these regions may interfere with the binding of UTR-binding proteins and/or translation initiation complexes.
2. Compare the potential target sites to the human genome database and eliminate from consideration any target sequences with significant homology to other coding sequences. The homology search can be performed using BLAST, which can be found on the NCBI server at: www.ncbi.nlm.nih.gov/BLAST/
3. Select qualifying target sequences for synthesis. On the website of Ambion, several preferable target sequences can be selected along the length of the gene for evaluation.

The siRNAs inhibit the expression of over-expressed OEX protein and is thereby useful for suppressing the biological activity of the protein. Therefore, a composition comprising the siRNA is useful in treating or preventing ovarian endometriosis.

The nucleic acids that inhibit one or more gene products of overexpressed genes also include ribozymes against the over-expressed gene(s) (OEX 1-97 and OEX 186-242).

The ribozymes inhibit the expression of over-expressed OEX protein and is thereby useful for suppressing the biological activity of the protein. Therefore, a composition comprising the ribozyme is useful in treating or preventing ovarian endometriosis.

Generally, ribozymes are classified into large ribozymes and small ribozymes. A large ribozyme is known as an enzyme that cleaves the phosphate ester bond of nucleic acids. After the reaction with the large ribozyme, the reacted site consists of a 5'-phosphate and 3'-hydroxyl group. The large ribozyme is further classified into (1) group I intron RNA catalyzing transesterification at the 5'-splice site by guanosine; (2) group II intron RNA catalyzing self-splicing through a two step reaction via lariat structure; and (3) RNA component of the ribonuclease P that cleaves the tRNA precursor at the 5' site through hydrolysis. On the other hand, small ribozymes have a smaller size (about 40 bp) compared to the large ribozymes and cleave RNAs to generate a 5'-hydroxyl group and a 2'-3' cyclic phosphate. Hammerhead type ribozymes (Koizumi et al. (1988) FEBS Lett. 228: 225) and hairpin type ribozymes (Buzayan (1986) Nature 323: 349; Kikuchi and Sasaki (1992) Nucleic Acids Res. 19: 6751) are included in the small ribozymes. Methods for designing and constructing ribozymes are known in the art (see Koizumi et al. (1988) FEBS Lett. 228: 225; Koizumi et al. (1989) Nucleic Acids Res. 17: 7059; Kikuchi and Sasaki (1992) Nucleic Acids Res. 19: 6751) and ribozymes inhibiting the expression of an over-expressed OEX protein can be constructed based on the sequence information of the nucleotide sequence encoding the OEX protein according to conventional methods for producing ribozymes.

The ribozymes inhibit the expression of over-expressed OEX protein and is thereby useful for suppressing the biological activity of the protein. Therefore, a composition comprising the ribozyme is useful in treating or preventing ovarian endometriosis.

Alternatively, the function of one or more gene products of the over-expressed genes is inhibited by administering a compound that binds to or otherwise inhibits the function of the gene products. For example, the compound is an antibody which binds to the over-expressed gene product or gene products.

The present invention refers to the use of antibodies, particularly antibodies against a protein encoded by an up-regulated marker gene, or a fragment of the antibody. As used herein, the term "antibody" refers to an immunoglobulin molecule having a specific structure that interacts (binds) specifically with a molecule comprising the antigen used for synthesizing the antibody *(i.e.,* the up-regulated marker gene product) or with an antigen closely related to it. An antibody that binds to the over-expressed OEX nucleotide may be in any form, such as monoclonal or polyclonal antibodies, and includes antiserum obtained by immunizing an animal such as a rabbit with the polypeptide, all classes of polyclonal and monoclonal antibodies, human antibodies and humanized antibodies produced by genetic recombination.

Furthermore, the antibody used in the method of treating or preventing ovarian endometriosis of the present invention may be a fragment of an antibody or a modified antibody, so long as it binds to one or more of the proteins encoded by the marker genes. For instance, the antibody fragment may be Fab, F(ab')2, Fv or single chain Fv (scFv), in which Fv fragments from H and L chains are ligated by an appropriate linker (Huston et al. (1988) Proc. Natl. Acad. Sci. USA 85: 5879-83). More specifically, an antibody fragment may be generated by treating an antibody with an enzyme, such as papain or pepsin. Alternatively, a gene encoding the antibody fragment may be constructed, inserted into an expression vector, and expressed in an appropriate host cell (see, for example, Co et al. (1994) J. Immunol. 152: 2968-76; Better M. and Horwitz (1989) Methods Enzymol. 178:476-96; Pluckthun and Skerra (1989) Methods Enzymol. 178: 497-515; Lamoyi (1986) Methods Enzymol. 121: 652-63; Rousseaux et al. (1986) Methods Enzymol. 121:663-9; Bird and Walker (1991) Trends Biotechnol. 9: 132-7).

An antibody may be modified by conjugation with a variety of molecules, such as polyethylene glycol (PEG). The modified antibody can be obtained by chemically modifying an antibody. These modification methods are conventional in the field.

Alternatively, an antibody may be obtained as a chimeric antibody, between a variable region derived from nonhuman antibody and the constant region derived from human antibody, or as a humanized antibody, comprising the complementarity determining region (CDR) derived from nonhuman antibody, the frame work region (FR) derived from human antibody, and the constant region. Such antibodies can be prepared using known technology.

The present invention provides a method for treating or preventing ovarian endometriosis, using an antibody against an over-expressed OEX polypeptide. According to the method, a pharmaceutically effective amount of an antibody against the OEX polypeptide is administered. An antibody against an over-expressed OEX polypeptide is administered at a dosage sufficient to reduce the activity of the OEX protein. Alternatively, an antibody binding to a cell surface marker specific for tumor cells can be used as a tool for drug delivery. Thus, for example, an antibody against an over-expressed OEX polypeptide conjugated with a cytotoxic agent may be administered at a dosage sufficient to injure tumor cells.

The present invention also relates to a method of treating or preventing ovarian endometriosis in a subject comprising administering to said subject a vaccine comprising a polypeptide encoded by a nucleic acid selected from the group consisting of OEX 1-97 and OEX 186-242 or an immunologically active fragment of said polypeptide, or a polynucleotide encoding the polypeptide or the fragment thereof. Administration of the polypeptide induces an anti-tumor immunity in a subject. The polypeptide or the immunologically active fragments thereof are useful as vaccines against ovarian endometriosis. An ovarian endometriosis which is benign tumor can be treated or prevented via inducing anti-tumor immunity in a subject. In some cases the proteins or fragments thereof may be administered in a form bound to the T cell recepor (TCR) or presented on an antigen presenting cell (APC), such as macrophage, dendritic cell (DC) or B-cells. Due to the strong antigen presenting ability of DC, the use of DC is most preferable among the APCs.

In the present invention, the phrase "vaccine against ovarian endometriosis" refers to a substance that has the function to induce anti-tumor immunity or immunity to suppress ovarian endometriosis upon inoculation into animals. In general, anti-tumor immunity includes immune responses such as follows:
- induction of cytotoxic lymphocytes against tumors,
- induction of antibodies that recognize tumors, and
- induction of anti-tumor cytokine production.

Therefore, when a certain protein induces any one of these immune responses upon inoculation into an animal, the protein is decided to have anti-tumor immunity inducing effect. The induction of the anti-tumor immunity by a protein can be detected by observing *in vivo* or *in vitro* the response of the immune system in the host against the protein.

For example, a method for detecting the induction of cytotoxic T lymphocytes is well known. A foreign substance that enters the living body is presented to T cells and B cells by the action of antigen presenting cells (APCs). T cells that respond to the antigen presented by APC in antigen specific manner differentiate into cytotoxic T cells (or cytotoxic T lymphocytes; CTLs) due to stimulation by the antigen, and then proliferate (this is referred to as activation of T cells). Therefore, CTL induction by a certain peptide can be evaluated by presenting the peptide to T cell by APC, and detecting the induction of CTL. Furthermore, APC has the effect of activating CD4+ T cells, CD8+ T cells, macrophages, eosinophils and NK cells. Since CD4+ T cells and CD8+ T cells are also important in anti-tumor immunity, the anti-tumor immunity inducing action of the peptide can be evaluated using the activation effect of these cells as indicators.

A method for evaluating the inducing action of CTL using dendritic cells (DCs) as APC is well known in the art. DC is a representative APC having the strongest CTL inducing action among APCs. In this method, the test polypeptide is initially contacted with DC and then this DC is contacted with T cells. Detection of T cells having cytotoxic effects against the cells of interest after the contact with DC shows that the test polypeptide has an activity of inducing the cytotoxic T cells. Activity of CTL against tumors can be detected, for example, using the lysis of ⁵¹Cr-labeled tumor cells as the indicator. Alternatively, the method of evaluating the degree of tumor cell damage using ³H-thymidine uptake activity or LDH (lactose dehydrogenase)-release as the indicator is also well known.

Apart from DC, peripheral blood mononuclear cells (PBMCs) may also be used as the APC. The induction of CTL is reported to be enhanced by culturing PBMC in the presence of GM-CSF and IL-4. Similarly, CTL has been shown to be induced by culturing PBMC in the presence of keyhole limpet hemocyanin (KLH) and IL-7.

The test polypeptides confirmed to possess CTL inducing activity by these methods are polypeptides having DC activation effect and subsequent CTL inducing activity. Therefore, polypeptides that induce CTL against tumor cells are useful as vaccines against ovarian endometriosis. Furthermore, APC that acquired the ability to induce CTL against ovarian endometriosis by contacting with the polypeptides are useful as vaccines against ovarian endometriosis. Furthermore, CTL that acquired cytotoxicity due to presentation of the polypeptide antigens by APC can be also used as vaccines against ovarian endometriosis. Such therapeutic methods for ovarian endometriosis using anti-tumor immunity due to APC and CTL are referred to as cellular immunotherapy.

Generally, when using a polypeptide for cellular immunotherapy, efficiency of the CTL-induction is known to increase by combining a plurality of polypeptides having different structures and contacting them with DC. Therefore, when stimulating DC with protein fragments, it is advantageous to use a mixture of multiple types of fragments.

Alternatively, the induction of anti-tumor immunity by a polypeptide can be confirmed by observing the induction of antibody production against tumors. For example, when antibodies against a polypeptide are induced in a laboratory animal immunized with the polypeptide, and when growth, proliferation or metastasis of tumor cells is suppressed by those antibodies, the polypeptide can be determined to have an ability to induce anti-tumor immunity.

Anti-tumor immunity is induced by administering the vaccine of this invention, and the induction of anti-tumor immunity enables treatment and prevention of ovarian endometriosis. Therapy against or prevention of the onset of ovarian endometriosis includes any of the steps, such as inhibition of the growth of endometrial cyst cells, involution of endometrial cyst cells and suppression of occurrence of endometrial cyst cells. Decrease in mortality of individuals having ovarian endometriosis, decrease of endometriosis markers in the blood, alleviation of detectable symptoms accompanying ovarian endometriosis and such are also included in the therapy or prevention of ovarian endometriosis. Such therapeutic and preventive effects are preferably statistically significant. For example, in observation, at a significance level of 5% or less, wherein the therapeutic or preventive effect of a vaccine against ovarian endometriosis is compared to a control without vaccine administration. For example, Student's t-test, the Mann-Whitney U-test or ANOVA may be used for statistical analyses.

The above-mentioned protein having immunological activity, or a polynucleotide or vector encoding the protein may be combined with an adjuvant. An adjuvant refers to a compound that enhances the immune response against the protein when administered together (or successively) with the protein having immunological activity. Examples of adjuvants include cholera toxin, salmonella toxin, alum and such, but are not limited thereto. Furthermore, the vaccine of this invention may be combined appropriately with a pharmaceutically acceptable carrier. Examples of such carriers are sterilized water, physiological saline, phosphate buffer, culture fluid and such. Furthermore, the vaccine may contain as necessary, stabilizers, suspensions, preservatives, surfactants and such. The vaccine is administered systemically or locally. Vaccine administration may be performed by single administration or boosted by multiple administrations.

When using APC or CTL as the vaccine of this invention, ovarian endometriosis can be treated or prevented, for example, by the *ex vivo* method. More specifically, PBMCs of the subject receiving treatment or prevention are collected, the cells are contacted with the polypeptide *ex vivo,* and following the induction of APC or CTL, the cells may be administered to the subject. APC can be also induced by introducing a vector encoding the polypeptide into PBMCs *ex vivo.* APC or CTL induced *in vitro* can be cloned prior to administration. By cloning and growing cells having high activity of damaging target cells, cellular immunotherapy can be performed more effectively. Furthermore, APC and CTL isolated in this manner may be used for cellular immunotherapy not only against individuals from whom the cells are derived, but also against similar types of diseases in other individuals.

### Pharmaceutical compositions for treating or preventing ovarian endometriosis

The present invention provides compositions for treating or preventing ovarian endometriosis comprising a compound selected by the present method of screening for a compound that alters the expression or activity of an ovarian endometriosis-associated gene. Such therapeutic compositions are administered throughout the menstrual cycle or concordantly with a specific phase, e.g., proliferative or secretory phase of the cycle and may be administered to humans and other mammals, such as mice, rats, guinea-pig, rabbits, cats, dogs, sheep, pigs, cattle, monkeys, baboons or chimpanzees. Pharmaceutical formulations of the present compositions include those suitable for oral, rectal, nasal, topical (including buccal and sub-lingual), vaginal or parenteral (including intramuscular, sub-cutaneous and intravenous) administration, or for administration by inhalation or insufflation. The formulations are optionally packaged in discrete dosage units.

Pharmaceutical formulations suitable for oral administration include capsules, cachets or tablets, each containing a predetermined amount of the active ingredient. Formulations also include powders, granules, solutions, suspensions or emulsions. The active ingredient is optionally administered as a bolus electuary or paste. Tablets and capsules for oral administration may contain conventional excipients such as binding agents, fillers, lubricants, disintegrant or wetting agents. A tablet may be made by compression or molding, optionally with one or more formulational ingredients. Compressed tablets may be prepared by compressing in a suitable machine the active ingredients in a free-flowing form such as a powder or granules, optionally mixed with a binder, lubricant, inert diluent, lubricating, surface active or dispersing agent. Molded tablets may be made via molding in a suitable machine a mixture of the powdered compound moistened with an inert liquid diluent. The tablets may be coated according to methods well known in the art. Oral fluid preparations may be in the form of, for example, aqueous or oily suspensions, solutions, emulsions, syrups or elixirs, or may be presented as a dry product for reconstitution with water or other suitable vehicle prior to use. Such liquid preparations may contain conventional additives such as suspending agents, emulsifying agents, non-aqueous vehicles (which may include edible oils) or preservatives. The tablets may optionally be formulated so as to provide slow or controlled release of the active ingredient *in vivo.* A package of tablets may contain one tablet to be taken on each of the month. The formulation or does of medicament varies with respect to the phase (proliferative or secretory) of the menstrual cycle.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit dose or multi-dose containers, for example sealed ampoules and vials, and may be stored in a freeze-dried (lyophilized) condition requiring only the addition of the sterile liquid carrier, for example, saline, water-for-injection, immediately prior to use. Alternatively, the formulations may be presented for continuous infusion. Extemporaneous injection solutions and suspensions may be prepared from sterile powders, granules and tablets of the kind previously described.

Formulations for rectal administration include suppositories with standard carriers such as cocoa butter or polyethylene glycol. Formulations for topical administration in the mouth, for example, buccally or sublingually, include lozenges, which contain the active ingredient in a flavored base such as sucrose and acacia or tragacanth, and pastilles comprising the active ingredient in a base such as gelatin, glycerin, sucrose or acacia. For intra-nasal administration of an active ingredient, a liquid spray or dispersible powder or in the form of drops may be used. Drops may be formulated with an aqueous or non-aqueous base also comprising one or more dispersing agents, solubilizing agents or suspending agents.

For administration by inhalation the compositions are conveniently delivered from an insufflator, nebulizer, pressurized packs or other convenient means of delivering an aerosol spray. Pressurized packs may comprise a suitable propellant such as dichlorodifluoromethane, trichlorofluoromethane, dichiorotetrafluoroethane, carbon dioxide or other suitable gas. In the case of a pressurized aerosol, the dosage unit may be determined by providing a valve to deliver a metered amount.

Alternatively, for administration by inhalation or insufflation, the compositions may take the form of a dry powder composition, for example, a powder mix of an active ingredient and a suitable powder base such as lactose or starch. The powder composition may be presented in unit dosage form in, for example, capsules, cartridges, gelatin or blister packs from which the powder may be administered with the aid of an inhalator or insufflators.

Other formulations include implantable devices and adhesive patches; which release a therapeutic agent.

When desired, the above described formulations, adapted to give sustained release of the active ingredient, may be employed. The pharmaceutical compositions may also contain other active ingredients such as antimicrobial agents, immunosuppressants or preservatives.

It should be understood that in addition to the ingredients particularly mentioned above, the formulations of this invention may include other agents conventional in the art having regard to the type of formulation in question, for example, those suitable for oral administration may include flavoring agents.

Preferred unit dosage formulations are those containing an effective dose, as recited below, of the active ingredient or an appropriate fraction thereof.

For each of the aforementioned conditions, the compositions, e.g., polypeptides and organic compounds are administered orally or via injection at a dose of from about 0.1 to about 250 mg/kg per day. The dose range for adult humans is generally from about 5 mg to about 17.5 g/day, preferably about 5 mg to about 10 g/day, and most preferably about 100 mg to about 3 g/day. Tablets or other unit dosage forms of presentation provided in discrete units may conveniently contain an amount which is effective at such dosage or as a multiple of the same, for instance, units containing about 5 mg to about 500 mg, usually from about 100 mg to about 500 mg.

The dose employed will depend upon a number of factors, including the age and sex of the subject, the precise disorder being treated, and its severity. Also the route of administration may vary depending upon the condition and its severity.

The present invention further provides a composition for treating or preventing ovarian endometriosis comprising active ingredient that inhibits the expression of any one of the gene selected from the group of OEX 1-97 or OEX 186-242. Such active ingredient can be an antisense-oligonucleotide, siRNA or ribozyme against the gene, or derivatives, such as expression vector, of the antisense-oligonucleotide, siRNA or ribozyme. The active ingredient may be made into an external preparation, such as liniment or a poultice, by mixing with a suitable base material which is inactive against the derivatives.

Also, as needed, the active ingredient can be formulated into tablets, powders, granules, capsules, liposome capsules, injections, solutions, nose-drops and freeze-drying agents by adding excipients, isotonic agents, solubilizers, preservatives, pain-killers and such. These can be prepared according to conventional methods for preparing nucleic acid containing pharmaceuticals.

Preferably, the antisense-oligonucleotide derivative, siRNA derivative or ribozyme derivative is given to the patient by direct application to the ailing site or by injection into a blood vessel so that it will reach the site of ailment. A mounting medium can also be used in the composition to increase durability and membrane-permiability. Examples of mounting mediums include liposome, poly-L-lysine, lipid, cholesterol, lipofectin and derivatives thereof.

The dosage of such compositions can be adjusted suitably according to the patient's condition and used in desired amounts. For example, a dose range of 0.1 to 100 mg/kg, preferably 0.1 to 50 mg/kg can be administered.

Another embodiment of the present invention is a composition for treating or preventing ovarian endometriosis comprising an antibody against a polypeptide encoded by any one of the genes selected from the group of OEX 1-97 and OEX 186-242 or fragments of the antibody that bind to the polypeptide.

Although there are some differences according to the symptoms, the dose of an antibody or fragments thereof for treating or preventing ovarian endometriosis is about 0.1 mg to about 100 mg per day, preferably about 1.0 mg to about 50 mg per day and more preferably about 1.0 mg to about 20 mg per day, when administered orally to a normal adult (weight 60 kg).

When administering parenterally, in the form of an injection to a normal adult (weight 60 kg), although there are some differences according to the condition of the patient, symptoms of the disease and method of administration, it is convenient to intravenously inject a dose of about 0.01 mg to about 30 mg per day, preferably about 0.1 to about 20 mg per day and more preferably about 0.1 to about 10 mg per day. Also, in the case of other animals too, it is possible to administer an amount converted to 60 kg of body-weight.

The following examples are presented to illustrate the present invention and to assist one of ordinary skill in making and using the same. The examples are not intended in any way to otherwise limit the scope of the invention.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. Any patents, patent applications and publications cited herein are incorporated by reference.

### Best Mode for Carrying out the Invention

The present invention is illustrated in details by following Examples, but is not restricted to these Examples.

### Materials and methods

### 1. Tissue preparation

Endometrial cysts were obtained after informed preoperative consent from 6 patients who underwent cystectomy at the Department of Obstetrics and Gynecology, the University of Tokyo Hospital. For immunohistochemistry, tissue sections were mounted in O.C.T compound (Sakura Finetechnical) immediately after resection.

### 2. Semi-quantitative RT-PCR

Semi-quantitative RT-PCR experiments were conducted as described previously (Ono et al. (2000) Cancer Res. 60: 5007-11). Endometrial cysts were obtained to extract total RNA, and T7-based RNA amplification with the total RNA was performed according to previous study (Arimoto et al. (2003) Int. J. Oncol. 22: 551-60). A 3-µg aliquot of amplified-RNA from each sample was reverse-transcribed for single-stranded cDNAs using random primer (Roche) and Superscript II (Life Technologies, Inc.). Each cDNA mixture was diluted for subsequent PCR amplification with these primer sets (TFPI-2 forward primer: 5'-TGACAGCATGAGGAAACAAATC-3'(SEQ.ID.NO.23); reverse primer: 5'-ACGACCCCAAGAAATGAGTG-3' (SEQ.ID.NO.24); ITLN forward primer: 5'-GCATTGGTGGAGGAGGATAC-3' (SEQ.ID.NO.25); reverse primer: 5'-TGCCATTAACATTCTAGCTACTGG-3' (SEQ.ID.NO.26); G3PDH forward primer: 5'-CGACCACTTTGTCAAGCTCA-3' (SEQ.ID.NO.21); reverse primer: 5'-GGTTGAGC ACAGGGTACTTTATT-3' (SEQ.ID.NO.22)). The expression of G3PDH served as an internal control. PCR reactions were optimized for the number of cycles to ensure product intensity within the linear phase of amplification.

### 3. Northern blotting

The Multiple-tissue Northern blot membranes containing 2-µg of poly(A)⁺ RNA from various human tissues (Clontech) were hybridized with ³²P-labeled partial cDNA fragments of TFPI-2 or ITLN (TFPI-2 forward primer: 5'-GGAAAATTCGGAAGAAGCAA-3' (SEQ.ID.NO.27); reverse primer: 5'-ACGACCCCAA GAAATGAGTG-3' (SEQ.ID.NO.24); ITLN forward primer: 5'-CGGGATTTGTTCAGTTCAGG-3' (SEQ.ID.NO.28); reverse primer: 5'-TGCCATTAACATTCTAGCTACTGG-3' (SEQ.ID.NO.26)): Conditions of hybridization and washing were described previously (Nakagawa et al. (2000) Oncogene 19: 210-6).

### 4. Recombinant protein production in Escherichia coli and polyclonal antibody generation

Recombinant proteins were prepared from the human TFPI-2 cDNA clone (GenBank Accession No. D29992) or ITLN (GenBank Accession No. BC020664) without encoding sequence of signal peptides (TFPI2: N-terminal 22 residues, ITLN: N-terminal 18 residues) by inserting into *Escherichia coli* expression vector pET28a (Novagen) and transforming into BL21-CodonPlus^{®} (DE3)-RIL competent cells (Stratagene). Protein expression was induced by 0.5mM isopropyl b-D-thiogalactoside (IPTG) with incubation at 37°C for 3 hr, and harvested by centrifugation. *E. coli* cell pellet that expressed recombinant TFPI-2 or ITLN were dissolved in 100mM sodium phosphate dehydrate (pH 8.0) containing 6M guanidine hydrochloride, 10mM Tris and 10mM imidazole. Histidine-tagged TFPI-2 or ITLN protein was purified by BD TALON^{™} Metal Affinity Resins (BD Biosciences). Then, 6 M guanidine hydrochloride was replaced with 8M urea. These purified recombinant proteins were refolded by diluting; the concentration of urea was reduced to 4M (TFPI-2) or 2.5M (ITLN). In addition, ITLN protein was further purified by anion-exchanged high performance liquid chromatography using a Mono-Q HR5/5 column (Amersham Biosciences) with ÄKTAexplorer 10S (Amersham Biosciences). These protein solutions were injected into rabbits every week, and after 10 times of immunization, antiserum was collected (MBL). The specific antibodies were isolated by affinity chromatography using Affi-Gel 10 (for TFPI-2, Bio-Rad) or Affi-Gel 15 (for ITLN, Bio-Rad) covalently bound to purify recombinant proteins.

### 5. Cell lines

Human endometrial adenocarcinoma HEC-151 cell line was provided from Kitasato University (Sagamihara, Japan) and was maintained in Eagle's minimal essential medium with 10% fetal bovine serum (FBS). Human glioma Hs.683 cell line was purchased from the American Type Culture Collection (Manassas, VA, USA). Hs.683 and Cos-7 cells were maintained in Dulbecco's modified Eagle's medium with 10% FBS. Cells were maintained at 37°C in an atmosphere of humidified air with 5% CO₂.

### 6. Western blotting

Samples were resolved by SDS-PAGE under reducing conditions and transferred onto Hybond^{™} ECL^{™} Nitrocellulose membranes (Amersham Pharmacia Biotech). The blotted membranes were blocked with Block Ace^{™} powder (Dainippon Seiyaku) and treated with rabbit anti-TFPI-2 (0.34 µg/ml) or anti-ITLN (0.16 µg/ml) specific polyclonal antibodies. After washing, the blots were treated with horseradish peroxidase-conjugated donkey anti-rabbit IgG (Amersham Biosciences) and developed with enhanced chemilumine- scence (ECL; Amersham Biosciences).

### 7. Immunofluorescent staining

Cells were replated on Lab- Tek^{®} II Chamber Slide System (Nalge Nunc International) followed by fixation with 4% paraformaldehyde in PBS and permeabilization with 0.1% Triton X-100 in PBS for 3 min at 4°C. After blocking with 3% BSA in PBS for 1 h at room temperature, the cells were incubated with rabbit anti-TFPI-2 (0.34 µg/ml) or anti-ITLN (0.16 µg/ml) antibodies for 1 h at room temperature. These antibodies were stained with a goat anti-rabbit secondary antibody conjugated to rhodamine, respectively, and viewed with a BX51 microscope (Olympus). As described in the following stable transformants section, stable transformants were also incubated with mouse anti-myc 9E10 monoclonal antibody (Santa Cruz Biotechnology, 0.2µg/ml) and stained with a rabbit anti-mouse secondary antibody conjugated to FITC.

### 8. Immunohistochemical staining and cross-inhibition assay

Achieved, paraformaldehyde-fixed, paraffin-embedded tissue sections were purchased from Biochain Institute, Inc. Sections were stained with DAKO EnVision^{™}+ System, HRP (DAB) (Dako) according to manufacturer's protocol. The polyclonal antibodies were used at 2.5 µg/ml (anti-TFPI-2 antibody) or at 2 µg/ml (anti-ITLN antibody). For immunostaining inhibition, antibodies were pre-incubated overnight at 4°C with the corresponding recombinant proteins that were used as the antigen (1.5 µg each).

### 9. Construction of stable transformants

Cos-7 cells were seeded and transfected with pcDNA3.1/myc-His^{©}(-)-TFPI-2 or pcDNA3.1/myc-His^{©}(-)-ITLN (Invitrogen), or with an empty vector as a control, using FuGENE6 Transfection Reagent (Roche), according to manufacturer's protocol. Cells were cultured for up to 3 weeks in the medium containing 0.4mg/ml of G418. Individual clones were isolated with cloning cylinders. The cell clones that expressed TFPI-2 or ITLN (as confirmed by RT-PCR, Western blotting, and immunofluorescent staining) were maintained in the medium containing 0.4mg/ml of G418 and used for further investigations.

### [Example 1] Correlation of clinicopathological features with differential gene expression in endometrial tissue of the patient

Endometrial cysts were obtained after informed pre-operative consent from 23 patients (women) who underwent cystectomy. Relevant clinical features of these patients are summarized in Table 10 below.

| Case No. | Age | Phase | Days after last menstrual period | Cyst size (cm) |
|---|---|---|---|---|
| 6 | 35 | proliferative | 9 | 2, 9 |
| 15 | 38 | proliferative | 10 | 4 |
| 19 | 44 | proliferative | 10 | 7 |
| 21 | 25 | proliferative | 1 | 5 |
| 22 | 37 | proliferative | 7 | 5, 7 |
| 302 | 35 | proliferative | 13 | 8 |
| 303 | 35 | proliferative | 15 | 6, 4 |
| 304 | 25 | proliferative | 9 | 6, 4 |
| 305 | 31 | proliferative | 4 | 7 |
| 3 | 37 | secretory | 21 | 5 |
| 7 | 32 | secretory | 23 | 8 |
| 11 | 23 | secretory | 19 | 6 |
| 12 | 29 | secretory | 31 | 7 |
| 14 | 41 | secretory | 12 | 8, 2 |
| 16 | 44 | secretory | 13 | 6 |
| 18 | 39 | secretory | 22 | 4 |
| 101 | 41 | secretory | 15 | 5 |
| 102 | 37 | secretory | 24 | 6 |
| 104 | 35 | secretory | 15 | 6 |
| 105 | 31 | secretory | 37 | 5, 6 |
| 106 | 31 | secretory | 35 | 6 |
| 107 | 30 | secretory | 30 | 3 |
| 151 | 38 | secretory | 25 | 9 |
| Two cysts for the samples in case No.6, 22, 303, 304, 14 and 105 were used. | | | | |

The ages of the patients ranged from 23 to 44, and none had been on hormonal therapy within two years prior to the surgery. Nine patients were in the proliferative phase of the menstrual cycle at the time of surgery and the other 14 were in the secretory phase. Cysts were histopathologically diagnosed according to standard methods. As control samples, eutopic endometrial tissues were obtained by dilatation and curettage from the uteri of 14 of the patients, seven in the proliferative phase and the other seven in the secretory phase. Epithelial cells were scraped immediately after resection and suspended in ice-cold Dulbecco's modified Eagle's medium (DMEM: Sigma) supplemented with 10% fetal calf serum (FCS), then separated from interstitial cells under a stereomicroscope, and their purity was confirmed under a phase-contrast microscope as described previously (Jimbo et al. (1997) Am. J. Pathol. 150: 1173-8). Isolation of epithelial cells was as described elsewhere (Hornung et al. (1998) Fertil. Steril. 69: 909-15; Sugawara et al. (1997) Biol. Reprod. 57: 936-42; Zhang et al. (1995) J. Cell Sci. 108: 323-32) with some modifications. Endometrial tissue was minced into small pieces and digested with 0.25% collagenase (Sigma) for 1 hour at 37°C. Using serial filtration, tissue debris was separated with 100-mm nylon sieves (Falcon) to remove mucus and undigested tissue. Then each filtrate was passed through a 40-mm nylon sieve that allowed stromal cells to go through. Epithelial glands were backwashed onto tissue culture dishes with DMEM/10%FCS and incubated at 37°C for 30 minutes in 5% CO₂ to attach fibroblasts on the dishes. Epithelial cells were recovered in the supernatant and used for extraction of total RNA.

### [Example 2] Identification of endometriosis-associated genes

Tissue obtained from diseased tissue (epithelial cells from endometriosis cysts) and normal tissues was evaluated to identify genes which are differently expressed in a disease state (endometriosis). The assays were carried out as follows.

### 1. RNA preparation and T7-based RNA amplification

Total RNA was extracted by suspending the epithelial cells in RNA lysis buffer (RLT buffer, QIAGEN Inc.) and purifying according to the manufacturer's instructions. After treatment with DNase I (Nippon Gene, Tokyo Japan), T7-based amplification was carried out according to known methods. Three rounds of amplification were performed to obtain sufficient amounts of amplified RNA (aRNA). Control samples were amplified in two rounds. Two kinds of universal control were prepared, one the eutopic aRNA mixture derived from seven patients in the proliferative phase and the other from seven patients in the secretory phase.

RNA amplified by this method accurately reflected the proportions in the original RNA source. This correspondence had been confirmed earlier by semi-quantitative reverse transcription-polymerase chain reaction (RT-PCR) experiments. As a result, data from microarrays proved to be consistent with results obtained by RT-PCR regardless whether the total RNA or aRNA was used as the template (Ono et al. (2000) Cancer Res. 60: 5007-11).

### 2. Preparation of the microarray

To obtain cDNAs for spotting on the glass slides, RT-PCR was performed for each gene as described previously (Okabe et al. (2001) Cancer Res. 61: 2129-2137). The PCR products were spotted on type 7 glass slides (Amersham Biosciences) with a Microarray Spotter Generation III (Amersham Biosciences). 4,608 genes were spotted in duplicate on each slide. Five different sets of slides were prepared (in total 23,040 genes), on each of which the same 52 housekeeping genes and two negative-control genes were spotted as well. Then 2.5-µg aliquots of aRNA from eutopic endometrial tissues and the corresponding ovarian endometrial cysts were labeled respectively with Cy3-dCTP and Cy5-dCTP (Amersham Biosciences). Hybridization, scanning and quantification of signals were performed as described previously (Ono et al. (2000) Cancer Res. 60: 5007-11) except that all processes were carried out with an Automated Slide Processor (Okabe et al. (2001) Cancer Res. 61: 2129-2137). The fluorescence intensities of Cy5 and Cy3 for each target spot were adjusted so that the mean Cy5/Cy3 ratios of 52 housekeeping genes were equal to one. Because data derived from low signal intensities are not very reliable, cut-off values for signal intensities on each slide were first determined to exclude genes for further analysis when both Cy3 and Cy5 dyes gave signal intensities lower than the cut-off. The relative expression of each gene (Cy5/Cy3 intensity ratio) was defined into one of four categories: up-regulated (ratio ≥ 2.0), down-regulated (ratio ≤ 0.5), unchanged (0.5 < ratio < 2.0) and not expressed (under the cutoff level of detection). Furthermore, the relative expression of each gene (Cy5/Cy3 intensity ratio) was defined into one of three additional categories: five fold up-regulated (ratio ≥ 5.0). 0.2 fold down-regulated (ratio ≤ 0.2) and 0.2-5 unchanged (0.2 < ratio, < 5.0).

### 3. Semi-quantitative RT-PCR

The representative 10 commonly up-regulated genes throughout the menstrual cycle were selected and examined for their expression levels by the semi-quantitative RT-PCR experiments. A 3-µg aliquot of aRNA from each sample was reverse-transcribed for single-stranded cDNAs using random primer (Roche) and Superscript II (Life Technologies, Inc.). Each cDNA mixture was diluted for subsequent PCR amplification with the same primer sets that were prepared for the target DNA- or G3PDH-specific reactions. The primer sequences are listed in Table 11.

| Genbank Accession No. | Abbreviation | Forward primer | SEQ ID NO : |
|---|---|---|---|
| M63262 | ALOX5AP | 5'-TGGGGTTGGTGTTCTCATCT-3' | 1 |
| AA583491 | HCA112 | 5'-TGGAATCTAGCCATGCCTCT-3' | 3 |
| X00457 | HLA-DPA1 | 5'-CTGAACTCCAGCTGCCCTAC-3' | 5 |
| K01505 | HLA-DQA1 | 5'-ATCGCCATCTACAGGAGCAG-3' | 7 |
| X63629 | CDH3 | 5'-ACCTTCTTAGGCCTCCTGGT-3' | 9 |
| X00637 | HP | 5'-CTGGTATGCGACTGGGATCT-3' | 11 |
| M81141 | HLA-DQB1 | 5'-TCCTGCACTGACTCCTGAGA-3' | 13 |
| M86511 | CD 14 | 5'-CCGAGGTGGATAACCTGACA-3' | 15 |
| M32093 | | 5'-CTCACACATTGCGAACAACA-3' | 17 |
| AI310156 | | 5'-GCCTCACAAAAGAGCCAGAG-3' | 19 |
| M33197 | G3PDH | 5'-CGACCACTTTGTCAAGCTCA-3' | 21 |
| | | Reverse Primer | |
| M63262 | ALOX5AP | 5'-ACCTGGTCACAAAACATCTTCAG-3' | 2 |
| AA583491 | HCA112 | 5'-ATCACATGACTACTCAGGAGGGG-3' | 4 |
| X00457 | HLA-DPA1 | 5'-GGAGAACAGAGGATAAAAGGCTC-3' | 6 |
| K01505 | HLA-DQA1 | 5'-CCAGGCATGTCTTTGTAGGTAAC-3' | 8 |
| X63629 | CDH3 | 5'-TACACGATTGTCCTCACCCTTC-3' | 10 |
| X00637 | HP | 5'-TGATTGACTCAGCAATGCAGG-3' | 12 |
| M81141 | HLA-DQB1 | 5'-GAATAGAAACAGAAACCCCTTGG-3' | 14 |
| M86511 | CD14 | 5'-GAATTGGTCGAAAAGTCCTCAAC-3' | 16 |
| M32093 | | 5'-ATGGTGCTTTTAAGAAGAGAGCC-3' | 18 |
| AI310156 | | 5'-GATCCACATTGGTGTTACCAGTT-3' | 20 |
| M33197 | G3PDH | 5'-GGTTGAGCACAGGGTACTTTATT-3' | 22 |

Accession numbers and gene symbols were retrieved from the Unigene Databases (build#131).

The expression of G3PDH served as an internal control. PCR reactions were optimized for the number of cycles to ensure product intensity within the linear phase of amplification.

### [Example 3] Identification of genes with clinically relevant expression patterns in ovarian endometrial cells

Gene-expression profiles of epithelial cells from ovarian endometrial cysts were analyzed using a comprehensive cDNA microarray system containing 23,040 genes. Individual data were excluded when Cy5 and Cy3 signals were under cut-off values (see Example 1). Then the expression levels in cysts from nine patients in the proliferative phase of the menstrual cycle were compared with the expression level in a universal control consisting of a mixture of eutopic endometrial cells from seven women in the same phase. For examining gene expression during the secretory phase, 14 relevant cysts were compared with a mixture of eutopic endometrial cells from seven women in that phase. When cut-off value of 2.0 was applied for the signal-intensity ratios of Cy5/Cy3, 15 genes including two expressed-tag sequences (ESTs) were up-regulated at least 70% of the 23 cases (Table 1). The protocol selected 42 genes including 15 ESTs as up-regulated only in the proliferative phase (Table 2), and 40 genes including 10 ESTs only in the secretory phase (Table 3). Most of the genes listed in Table 2 were also over-expressed in the secretory phase and most of the genes listed in Table 3 were also over-expressed in the proliferative phase, but both in fewer than 70% of the cases. However, some genes were up-regulated in exclusively in one phase or the other: S100 calcium-binding protein A13 (S100A13), myosin regulatory light chain 2 and smooth muscle isoform (MYRL2) were up-regulated specifically in the proliferative phase, while genes encoding four and a half LIM domains 2 (FHL2) and TSPY-like (TSPYL) were up-regulated in only the secretory phase. Many ectopic endometria histopathologically show proliferative features when their corresponding eutopic endometrial tissues are in the secretory phase (Mathur et al. (1990) Fertil. Steril. 54: 56-63; Molitor (1971) Am. J. Obstet. Gynecol. 110: 275-84). Endometrial cells might continue to proliferate throughout the menstrual cycle when the expression of some genes is altered.

Experiments also revealed down-regulation of numerous genes in the cysts. Three hundred thirty-seven genes, including one hundred sixty-four ESTs, were commonly under-expressed in more than 70% of the patients during either proliferative or secretory phases when the cut-off signal intensity ratio of Cy5/Cy3 was set to 0.5. One hundred forty-four other genes were selected, including forty-one ESTs, as being under-expressed only in the proliferative phase, and eight hundred thirty-five genes including four hundred twenty-eight ESTs only in the secretory phase. Among them, only the genes listed in Tables 4, 5 and 6 are those whose Cy5/Cy3 signal intensity ratios were less than 0.3 in more than 70% of the cases examined.

Semi-quantitative RT-PCR experiments were performed to confirm the differences in expression indicated by microarray analysis. By comparing the results with ratios of expression levels of the representative ten genes that were up-regulated throughout the menstrual cycle, good correspondence was verified with the microarray analysis in the great majority of cases tested (Fig. 1).

Many of the up-regulated genes encode elements of the immune system. For example, genes encoding histocompatibility proteins HLA-DPA1, HLA-DQA1, HLA-DQB1, HLA-DRA, HLA-DRB1 and CD 14 were all over-expressed throughout the menstrual cycle. In addition, genes encoding complement factors C3, BF, C1S, C1R and C2, as well as CEBPD and HLA-F, were up-regulated in cysts mainly during the secretory phase. Over-expression of those genes leads to endometriosis and to poor reproductive and production of autoantibodies.

As a clinical entity, endometriosis appears to be an estrogen-dependent disease whose major symptoms are dysmenorrhea, dyspareunia, chronic pelvic pain and infertility. The results of the microarray analysis indicated concordance with these clinical features. For example, high expression of complement components indicates that pelvic pain results from severe inflammation in the endometriotic lesion. For example, the gene encoding ALOX5AP, which is required for leukotriene biosynthesis, was up-regulated in 22 of the 23 patients examined (Fig. 2). Progressive inflammation or adhesion induced by over-expression of these genes causes physical damage to tubes and ovaries, leading to infertility. Furthermore, local intraperitoneal inflammation generates ascites. Peritoneal fluid in patients with endometriosis reduces fertility by inhibiting fimbrial capability of the cumulus-oocyte complex, movement of sperm, and growth of the embryo.

One of the up-regulated genes is TGFBI. The product of this gene inhibits natural killer activity while inducing angiogenesis and proliferation of endometrial stromal cells. In addition, TGFBI significantly inhibits development of early mice embryos. Increased expression of TGFBI in endometriotic tissue is involved in the progression of endometriosis and in infertility.

The down-regulation of oviductal glycoprotein 1 (OVGP1), observed in endometrial cysts during both phases of the menstrual cycle, is also noteworthy in relation to infertility. OVGP1 plays a role in protecting the early embryo and the fallopian tube from extracellular environments that are potentially noxious, such as peritoneal fluid, retrograde reflux of menstrual fluid, microorganisms and spermatozoa. Lowered expression of OVGP1 therefore may contribute to infertility in patients with endometriosis.

Down-regulation of tumor suppressor TP53 in cysts from ten of the 14 patients was detected in the secretory menstrual phase and in cysts from six of the nine patients in the proliferative phase. TP53BP2 was also under-expressed in seven of the nine cases in the proliferative phase. Although it is considered a benign disorder, endometriosis exhibits tumor-like features that include cellular proliferation, cellular invasion and neoangiogenesis. A significantly higher risk of ovarian cancer is associated with endometriosis (standardized incidence ratio = 1.9), especially among women with ovarian endometriosis of more than 10 years' duration (ratio = 4.2) (Brinton et al. (1997) Am. J. Obstet. Gynecol. 176: 572-9).

The differential gene expression patterns described herein are useful to diagnose endometrioid and clear-cell carcinoma of the ovary. In one study about 39.2% of patients with ovarian clear-cell carcinomas and 21.2% of patients with ovarian endometrioid carcinomas were affected with ovarian endometriosis (Yoshikawa et al. (2000) Gynecol. Obest. Invest. 50: 11-7). Shimizu et al. have noted that clear-cell carcinomas of the ovary tend to show negative expression of p53 (Shimizu et al. (1999) Cancer 85: 669-77). The data described herein also indicate that under-expression of TP53 and/or TP53BP2 is involved in "malignant" endometriosis. Furthermore, GADD34, GADD45A and GADD45B, proteins associated with apoptosis (DeSmaele et al. (2001) Nature 414: 308-13), were also down-regulated in the cysts in spite of the fact that transcriptional levels of those genes tend to increase under stressful conditions of growth-arrest or following treatment with DNA-damaging agents. Moreover, PIG11, which generates or responds to oxidative stress and has a role in p53-dependent apoptosis, was down-regulated in 11 of the 14 cases examined in the secretory phase and in six of the nine cases in the proliferative phase. Hence, decreased expression of these genes in endometrial epithelial cells affects apoptotic signals and may be associated with the tumor-like character of this disease.

Synthesis of ribosomal proteins increases in response to estrogen (Knowles (1978) Biochem. J. 170: 181-3; Muller and Knowles (1984) FEBS Lett. 174: 253-7), and microarray analyses have shown up-regulation of ribosomal protein S23 (RPS23) in ectopic endometrium (Eyster et al. (2002) Fertil. Steril. 77: 38-42). The data described herein revealed up-regulation of RPS11 and RPL11 as well as RPS23. The increase in expression may be a consequence of increased estrogen levels in endometriotic tissue.

When cut-off value of 5.0 was applied for the signal-intensity ratios of Cy5/Cy3, 20 genes including four expressed-tag sequences (ESTs) were found to be "5 fold up-regulated" in at least 50% of the 23 cases examined (Table 7). The protocol selected 38 genes including 19 ESTs as "5 fold up-regulated" only in the proliferative phase (Table 8) and 35 genes including 10 ESTs only in the secretory phase (Table 9). Most of the genes listed in Table 8 were also over-expressed in the other phase *(i.e.,* the secretory phase) and most of the genes listed in Table 9 were also over-expressed in the proliferative phase, but both in fewer than 50% of the cases. However, some genes were "5 fold up-regulated" in exclusively in one phase or the other: e.g., secreted frizzled-related protein 4 (SFRP4) and tissue factor pathway inhibitor 2 (TFPI2) were "5 fold up-regulated" specifically in the secretory phase.

The experiments also revealed down-regulation of numerous genes in the cysts; 36 genes, including 14 ESTs, were commonly under-expressed in more than 50% of the patients during either proliferative or secretory phases when the cut-off signal intensity ratio of Cy5/Cy3 was set to 0.2. Forty seven other genes were selected, including 13 ESTs, as being under-expressed only in the proliferative phase, and 276 genes including 156 ESTs only in the secretory phase.

Many of the "5 fold up-regulated" genes encode elements of the immune system; for example HLA-DRA, IGHG3, IGLλ, C1R and CD163 were all over-expressed throughout the menstrual cycle. In addition, genes encoding complement factors C3, BF, C1S and C2, as well as CEBPD and HLA-DQB1, were "5 fold up-regulated" in cysts mainly during the secretory phase.

As a clinical entity endometriosis appears to be an estrogen-dependent disease, the major symptoms of which are dysmenorrhea, dyspareunia, chronic pelvic pain and infertility. The results of the microarray analysis indicated concordance with these clinical features; e.g., high expression of complement components suggests that pelvic pain could result from severe inflammation in the endometriotic lesion. The correlation is supported by data indicating that the gene encoding ALOX5AP, which is required for leukotriene biosynthesis, was "5 fold up-regulated" in 21 of the 23 patients examined.

The nucleic acid sequences identified as "5 fold up-regulated" genes are useful as target of therapeutic agents for alleviating ovarian endometriosis or a predisposition to developing ovarian endometriosis.

### [Example 4] Confirmation of expression of TFPI-2 and ITLN in endometriosis by semi-quantitative RT-PCR

A cDNA microarray was used to analyze gene-expression profiles of 23,040 genes in ovarian endometrial cysts from 23 patients (Arimoto et al. (2003) Int. J. Oncol. 22: 551-60). Among the up-regulated genes, a gene, TFPI-2, which was overexpressed in all of 9 informative cases whose signal intensities of the gene were higher than the cut-off in the secretory phase and was down-regulated in more than 50% of the cases in the proliferative phase was focused and selected for further studies. In addition, ITLN, which was also up-regulated in 8 of 9 informative cases in the secretory phase and was up-regulated in all of 3 informative cases in the proliferative phase was also focused and selected for further studies. Furthermore, semi-quantitative RT-PCR analysis was performed to confirm elevated expression of TFPI-2 in the secretory phase and that of ITLN throughout the menstrual cycle in endometriosis (Fig. 3).

To investigate whether TFPI-2 and ITLN are secreted proteins as described previously, a population of mammalian cells that stably over-express these proteins were established by transfecting pcDNA3.1(-)-*TFPI*-*2*-myc-his or pcDNA3.1(-)-*ITLN-myc-*his into cos7 cells, and then the expression and subcellular localization in some transformants was confirmed by Western blotting (Fig. 4A) and immunofluorescent staining (Fig. 4B). As described previously, prominent TFPI-2 triplet bands (approximately 33, 31, 27 kDa) were observed from the TFPI-2 sense transformants with anti-TFPI-2 polyclonal antibody, and corresponding TFPI-2 proteins were also detected in culture medium (Sense), but not from the vector transfectants (Mock) (Fig. 4A; left panel). In addition, endogenous expression of TFPI-2 protein in HEC-151 and Hs.683 cells detected by Western blotting (Fig. 4A, right panel) and immunofluorescent staining (Fig. 4C) with anti-TFPI-2 polyclonal antibody revealed that TFPI-2 protein localizes in the cytoplasm such as secreted proteins. Similarly, a 40 kDa single band of ITLN was also observed in culture medium of the ITLN sense transformants with anti-ITLN polyclonal antibody (Fig.3A), and ITLN protein was observed to be localized in cytoplasm as well as the TFPI-2 (Fig. 4B). These results suggest that bothTFPI-2 and ITLN are secreted proteins.

### [Example 5] Detection of TFPI-2 and ITLN protein expression in endometrial cysts and normal human tissues by immunohistochemistry

TFPI-2 was initially demonstrated as being specifically expressed in the placenta (Fig. 5, upper panel), and ITLN in the colon, and to a less degree in the heart, small intestine, thymus, testis and spleen (Fig. 5, lower panel) by Northern blot analyses. Next, immunohistochemical staining on human normal tissues was carried out, and TFPI-2 protein was demonstrated to be expressed in syncytiotrophoblasts and decidual cells of placenta, but much weaker in the cytoplasm of hepatocytes around the vein and cardiac muscle cells, and almost no staining was observed in brain, kidney, lung and skeletal muscle (Fig. 6A). On the other hand, positive staining for ITLN protein was observed in the basal cells of crypts and mucosal epithelial cells of colon and small intestine while the cytoplasm of cardiac muscle cells was stained weaker and brain, kidney, liver and lung was not stained at all (Fig. 6B).

Furthermore, to investigate whether TFPI-2 or ITLN antibodies can specifically recognize the corresponding proteins in placenta or in small intestine, a cross-inhibition assay was performed. As a result, reduced staining of these cells showed that both polyclonal antibodies reacted specifically to the corresponding protein (Fig. 6C).

Moreover, the expression of TFPI-2 and ITLN proteins in the sections of endometrial cysts was investigated by immunohistochemistry. Positive staining for both proteins were observed on epithelial cells of endometrial cysts by anti-TFPI-2 antibody or anti-ITLN-antibody (Fig. 7, left and middle panels), whereas no positive staining in the same enodometrial cysts tissues could be observed using anti-rabbit IgG as the negative control.

### Industrial Applicability

Various gynecological studies about TFPI-2 were performed in terms of progression of pregnancy, but there is no report that this protein relates to the development of endometriosis. Further, little is known whether ITLN participates in the progression or maintenance of various gynecologic disorders or tumor disease. Therefore, the present inventors examined whether both of TFPI-2 and ITLN are secreted through the establishment of stable transformants of each gene, and demonstrated that these genes were overexpressed in endometrial cysts by semi-quantitative RT-PCR and immunohistochemistry (Fig. 3 and 7).

In conclusion, the present invention demonstrated possible involvement of TFPI-2 and ITLN proteins in human carcinogenesis. Since the expression of these transcripts is relatively low in normal human adult tissues, these genes themselves might serve as novel targets for therapy.

The gene-expression analysis of ovarian endometriosis described herein, obtained through a combination of laser-capture dissection and genome-wide cDNA microarray, has identified specific genes as targets for prevention and therapy of ovarian endometriosis. Based on the expression of a subset of these differentially expressed genes, the present invention provides molecular diagnostic markers for identifying or detecting ovarian endometriosis.

The methods described herein are also useful in the identification of additional molecular targets for prevention, diagnosis and treatment of ovarian endometriosis. The data reported herein add to a comprehensive understanding of ovarian endometriosis, facilitate development of novel diagnostic strategies and provide clues for identification of molecular targets for therapeutic drugs and preventative agents. Such information contributes to a more profound understanding of carcinogenesis, and provides indicators for developing novel strategies for diagnosis, treatment and ultimately prevention of ovarian endometriosis.

While the invention has been described in detail and with reference to specific embodiments thereof, it will be apparent to one skilled in the art that various changes and modifications can be made therein without departing from the spirit and scope of the invention.
1. A method of diagnosing ovarian endometriosis or a predisposition of developing ovarian endometriosis in a subject, comprising determining the expression level of an ovarian endometriosis-associated gene in a subject-derived biological sample, wherein an increase or decrease of said expression level compared to a control level of said gene indicates that said subject suffers from or is at risk of developing ovarian endometriosis.
2. The method of item 1, wherein said ovarian endometriosis-associated gene is selected from the group consisting of OEX 1-97 and 186-242, wherein an increase in said level compared to a normal control level indicates said subject suffers from or is at risk of developing ovarian endometriosis.
3. The method of item 2, wherein said increase is at least 10% greater than said normal control level.
4. The method of item 1, wherein said ovarian endometriosis-associated gene is selected from the group consisting of OEX 98-185, wherein a decrease in said level compared to a normal control level indicates said subject suffers from or is at risk of developing ovarian endometriosis.
5. The method of item 4, wherein said decrease is at least 10% lower than said normal control level.
6. The method of item 1, wherein said method further comprises determining the expression level of a plurality of the ovarian endometriosis-associated genes.
7. The method of item 1, wherein the expression level is determined by any one method selected from the group consisting of:
   (a) detecting the mRNA of an ovarian endometriosis-associated gene;
   (b) detecting the protein encoded by an ovarian endometriosis-associated gene; and
   (c) detecting the biological activity of the protein encoded by an ovarian endometriosis-associated gene.
8. The method of item 7, wherein said expression level is determined by detecting hybridization of an ovarian endometriosis-associated gene probe to a gene transcript in said subject-derived biological sample.
9. The method of item 8, wherein said hybridization step is carried out on a DNA array.
10. The method of item 1, wherein said biological sample is a tissue sample and comprises an epithelial cell.
11. The method of item 1, wherein said biological sample is a tissue sample comprising an endometrial cyst cell.
12. The method of item 11, wherein said biological sample is a tissue sample comprising an epithelial cell from an endometrial cyst.
13. An ovarian endometriosis reference expression profile, comprising a pattern of gene expression of two or more genes selected from the group consisting of OEX 1-242.
14. An ovarian endometriosis reference expression profile, comprising a pattern of gene expression of two or more genes selected from the group consisting of OEX 1-97 and 86-242.
15. An ovarian endometriosis reference expression profile, comprising a pattern of gene expression of two or more genes selected from the group consisting of OEX 98-185.
16. A method of screening for a compound that alters the expression of an ovarian endometriosis-associated gene, comprising the steps of
   (a) contacting a test cell that expresses an ovarian endometriosis-associated gene with a test compound;
   (b) determining the expression level of said ovarian endometriosis-associated gene; and
   (c) selecting the compound that alters the expression level compared to that in the absence of the test compound.
17. The method of item 16, wherein said the test cell is an epithelial cell.
18. The method of item 17, wherein said epithelial cell is isolated from an endometrial cyst.
19. The method of item 17, wherein the ovarian endometriosis-associated gene is selected from the group consisting of OEX 1-97 and 186-242, and a compound that decreases the expression level of said ovarian endometriosis-associated gene is selected in step (c).
20. The method of item 17, wherein the ovarian endometriosis-associated gene is selected from the group consisting of OEX 98-185, and a compound that increases the expression level of said ovarian endometriosis-associated gene is selected in step (c).
21. A method of screening for a compound that alters the expression of an ovarian endometriosis-associated gene, comprising the steps of
   (a) contacting a test compound with a cell into which a vector comprising a reporter gene linked downstream of a transcriptional regulatory region of an ovarian endometriosis-associated gene has been introduced;
   (b) measuring the activity of the reporter gene; and
   (c) selecting a compound that alters the expression level of the reporter gene compared to that in the absence of the test compound.
22. The method of item 21, wherein the ovarian endometriosis-associated gene is selected from the group consisting of OEX 1-97 and 186-242, and a compound that increases the activity of the reporter gene is selected in step (c).
23. The method of item 21, wherein the ovarian endometriosis-associated gene is selected from the group consisting of OEX 98-185, and a compound that decreases the activity of the reporter gene is selected in step (c).
24. A method of screening for a compound that alters the activity of an ovarian endometriosis-associated gene, comprising the steps of
   (a) contacting a test compound with a polypeptide encoded by an ovarian endometriosis-associated gene;
   (b) detecting the binding activity between the polypeptide and the test compound; and
   (c) selecting the compound that binds to the polypeptide.
25. A method of screening for a compound that alters the activity of an ovarian endometriosis-associated gene, comprising the steps of
   (a) contacting a test compound with a polypeptide encoded by an ovarian endometriosis-associated gene;
   (b) detecting the biological activity of the polypeptide; and
   (c) selecting the compound that alters the biological activity of the polypeptide in comparison with the biological activity detected in the absence of the test compound.
26. The method of item 25, wherein the ovarian endometriosis-associated gene is selected from the group consisting of OEX 1-97 and 186-242, and a compound that increases the biological activity of the polypeptide encoded by said ovarian endometriosis-associated gene is selected in step (c).
27. The method of item 26, wherein the ovarian endometriosis-associated gene is selected from the group consisting of OEX 98-185, and a compound that decreases the biological activity of the polypeptide encoded by said ovarian endometriosis-associated gene is selected in step (c).
28. A kit comprising one or more detection reagents which binds to one or more nucleic acid sequences selected from the group consisting of OEX 1-242.
29. An array comprising one or more nucleic acids which bind to one or more nucleic acid sequences selected from the group consisting of OEX 1-242.
30. A method of treating or preventing ovarian endometriosis in a subject comprising the step of inhibiting the expression of a gene selected from the group of OEX 1-97 and 186-242 in the subject or the activity of a polypeptide encoded by the gene.
31. The method of item 30, wherein the expression of the gene is inhibited by administering to said subject an antisense composition that comprises a nucleotide sequence complementary to a coding sequence selected from the group consisting of OEX 1-97 and OEX 186-242.
32. The method of item 30, wherein the expression of the gene is inhibited by administering to said subject a siRNA composition that reduces the expression of a nucleic acid sequence selected from the group consisting of OEX 1-97 and 186-242.
33. The method of item 30, wherein the activity of a the polypeptide encoded by the gene is inhibited by administering to said subject a pharmaceutically effective amount of an antibody or fragment thereof that bind to a polypeptide encoded by any one of the gene selected from the group consisting of OEX 1-97 and 186-242.
34. A method of treating or preventing ovarian endometriosis in a subject comprising the step of administering to said subject a vaccine comprising a polypeptide encoded by a nucleic acid sequence selected from the group consisting of OEX 1-97 and OEX 186-242 or an immunologically active fragment of said polypeptide.
35. A method of treating or preventing ovarian endometriosis in a subject comprising the step of administering to said subject a compound that decreases the expression or activity of a polypeptide encoded by a gene selected from the group of OEX 1-97 and OEX 186-242.
36. A method of treating or preventing ovarian endometriosis in a subject comprising the step of administering to said subject a compound that increases the expression or activity of a polypeptide encoded by a gene selected from the group of OEX 98-185.
37. A composition for treating or preventing ovarian endometriosis, said composition comprising a pharmaceutically effective amount of polynucleotide select from group consisting of OEX 98-185, or polypeptide encoded by thereof.
38. A composition for treating or preventing ovarian endometriosis, said composition comprising a pharmaceutically effective amount of an antisense-oligonucleotide or small interfering RNA against a nucleic acid sequence selected from the group consisting of OEX 1-97 and OEX 186-242.
39. A composition for treating or preventing ovarian endometriosis, said composition comprising a pharmaceutically effective amount of an antibody or fragment thereof that binds to a polypeptide encoded by any one of the gene selected from the group consisting of OEX 1-97 and OEX 186-242.
40. A composition for treating or preventing ovarian endometriosis, said composition comprising a pharmaceutically effective amount of the compound selected by the method of any one of items 16-26.

## Claims

1. A method of diagnosing ovarian endometriosis or a predisposition of developing ovarian endometriosis in a subject, comprising determining the expression level of an ovarian endometriosis-associated gene in a subject-derived biological sample, wherein an increase or decrease of said expression level compared to a control level of said gene indicates that said subject suffers from or is at risk of developing ovarian endometriosis.

2. The method of claim 1, wherein said ovarian endometriosis-associated gene is selected from the group consisting of OEX 228, 1-97, 186-227, 229-233 and 235-242, wherein an increase in said level compared to a normal control level indicates said subject suffers from or is at risk of developing ovarian endometriosis.

3. The method of claim 2, wherein said increase is at least 10% greater than said normal control level.

4. The method of claim 1, wherein said ovarian endometriosis-associated gene is selected from the group consisting of OEX 98-185, wherein a decrease in said level compared to a normal control level indicates said subject suffers from or is at risk of developing ovarian endometriosis.

5. The method of claim 4, wherein said decrease is at least 10% lower than said normal control level.

6. The method of claim 1, wherein the expression level is determined by any one method selected from the group consisting of:
(a) detecting the mRNA of an ovarian endometriosis-associated gene;
(b) detecting the protein encoded by an ovarian endometriosis-associated gene; and
(c) detecting the biological activity of the protein encoded by an ovarian endometriosis-associated gene.

7. The method of claim 6, wherein said expression level is determined by detecting hybridization of an ovarian endometriosis-associated gene probe to a gene transcript in said subject-derived biological sample.

8. The method of claim 7, wherein said hybridization step is carried out on a DNA array.

9. The method of claim 1, wherein said biological sample is a tissue sample and comprises
(a) an epithelial cell; or
(b) an endometrial cyst cell.

10. The method of claim 9, wherein said biological sample is a tissue sample comprising an epithelial cell from an endometrial cyst.

11. An in vitro method of screening for a compound that alters the expression of an ovarian endometriosis-associated gene, comprising the steps of
(a) contacting a test cell that expresses an ovarian endometriosis-associated gene with a test compound;
(b) determining the expression level of said ovarian endometriosis-associated gene; and
(c) selecting the compound that alters the expression level compared to that in the absence of the test compound.

12. The method of claim 11, wherein said the test cell is an epithelial cell.

13. The method of claim 12, wherein said epithelial cell is isolated from an endometrial cyst.

14. The method of claim 12, wherein the ovarian endometriosis-associated gene is selected from the group consisting of OEX 228, 1-97, 186-227, 229-233 and 235-242, and a compound that decreases the expression level of said ovarian endometriosis-associated gene is selected in step (c).

15. The method of claim 11, wherein the ovarian endometriosis-associated gene is selected from the group consisting of OEX 98-185, and a compound that increases the expression level of said ovarian endometriosis-associated gene is selected in step (c).

16. An in vitro method of screening for a compound that alters the expression of an ovarian endometriosis-associated gene, comprising the steps of
(a) contacting a test compound with a cell into which a vector comprising a reporter gene linked downstream of a transcriptional regulatory region of an ovarian endometriosis-associated gene has been introduced;
(b) measuring the activity of the reporter gene; and
(c) selecting a compound that alters the expression level of the reporter gene compared to that in the absence of the test compound.

17. The method of claim 16, wherein the ovarian endometriosis-associated gene is selected from the group consisting of OEX 228, 1-97, 186-227, 229-233 and 235-242, and a compound that decreases the activity of the reporter gene is selected in step (c).

18. The method of claim 16, wherein the ovarian endometriosis-associated gene is selected from the group consisting of OEX 98-185, and a compound that increases the activity of the reporter gene is selected in step (c).

19. A method of screening for a compound that alters the activity of an ovarian endometriosis-associated gene selected from the group consisting of OEX 228, 1-227, 229-233 and 235-242, comprising the steps of
(a) contacting a test compound with the polypeptide encoded by the gene;
(b) detecting the binding activity between the polypeptide and the test compound; and
(c) selecting the compound that binds to the polypeptide.

20. A method of screening for a compound that alters the activity of an ovarian endometriosis-associated gene, comprising the steps of
(a) contacting a test compound with a polypeptide encoded by an ovarian endometriosis-associated gene;
(b) detecting the biological activity of the polypeptide; and
(c) selecting the compound that alters the biological activity of the polypeptide in comparison with the biological activity detected in the absence of the test compound.

21. The method of claim 20, wherein the ovarian endometriosis-associated gene is selected from the group consisting of OEX 228, 1-97, 186-227, 229-233 and 235-242 and a compound that decreases the biological activity of the polypeptide encoded by said ovarian endometriosis-associated gene is selected in step (c).

22. The method of claim 20, wherein the ovarian endometriosis-associated gene is selected from the group consisting of OEX 98-185, and a compound that increases the biological activity of the polypeptide encoded by said ovarian endometriosis-associated gene is selected in step (c).

23. A kit comprising one or more detection reagents which binds to one or more nucleic acid sequences selected from the group consisting of OEX 228, 1-227, 229-233 and 235-242.

24. An array comprising one or more nucleic acids which bind to one or more nucleic acid sequences selected from the group consisting of OEX 228, 1-227, 229-233 and 235-242.

25. A pharmaceutical composition for treating or preventing ovarian endometriosis, said composition comprising
(a) an antisense-oligonucleotide or a small interfering RNA that reduces the expression of a nucleic acid sequence selected from the group consisting of OEX 228, 1-97, 186-227, 229-233 and 235-242;
(b) an antibody or fragment thereof that binds to a polypeptide encoded by a gene selected from the group consisting of OEX 228, 1-97, 185-227, 229-233 and 235-242; or
(c) a polynucleotide selected from the group consisting of OEX 98-185, or a polypeptide encoded thereby.

26. Use of
(a) an antisense-oligonucleotide or small interfering RNA that reduces the expression of a nucleic acid sequence selected from the group consisting of OEX 228, 1-97, 186-227, 229-233 and 235-242;
(b) an antibody or fragment thereof that binds to a polypeptide encoded by a gene selected from the group consisting of OEX 228, 1-97, 186-227, 229-233 and 235-242;
(c) a polynucleotide selected from the group consisting of OEX 98-185 or a polypeptide encoded thereby; or
(d) a polypeptide encoded by an ovarian endometriosis-associated gene selected from the group consisting of OEX 228,1-97,186-227, 229-233 and 235-242, or an immunologically active fragment of said polypeptide,
for the preparation of a pharmaceutical composition for treating or preventing ovarian endometriosis.


SEQUENCE LISTING

<110> ONCOTHERAPY SCIENCE, INC.

<120> Method of diagnosing ovarian endometriosis

<130> L1338 EP/1 S3

<150> US 60/407,365
<151> 2002-08-30

<150> US 60/450,920
<151> 2003-02-28

<160> 28

<170> PatentIn version 3.1

<210> 1
<211> 20
<212> DNA
<213> Artificial Sequence

<220>
<221> source
<223> /note="Description of Artificial Sequence: Artificially synthesized

primer sequence for RT-PCR"

<400> 1
tggggttggt gttctcatct 20

<210> 2
<211> 23
<212> DNA
<213> Artificial Sequence

<220>
<221> source
<223> /note="Description of Artificial Sequence: Artificially synthesized
primer sequence for RT-PCR"

<400> 2
acctggtcac aaaacatctt cag 23

<210> 3
<211> 20
<212> DNA
<213> Artificial Sequence

<220>

<221>  source
<223>  /note="Description of Artificial Sequence: Artificially synthesized
primer sequence for RT-PCR"

<400>  3
tggaatctag ccatgcctct                                                    20

<210>  4
<211>  23
<212>  DNA
<213>  Artificial Sequence

<220>
<221>  source
<223>  /note="Description of Artificial Sequence: Artificially synthesized
primer sequence for RT-PCR"

<400>  4
atcacatgac tactcaggag ggg                                                23

<210>  5
<211>  20
<212>  DNA
<213>  Artificial Sequence

<220>

<221>  source

<223>  /note="Description of Artificial Sequence: Artificially synthesized

primer sequence for RT-PCR"

<400>  5

ctgaactcca gctgccctac                                                    20

<210>  6

<211>  23

<212>  DNA

<213>  Artificial Sequence

<220>

<221>  source

<223>  /note="Description of Artificial Sequence: Artificially synthesized

primer sequence for RT-PCR"

<400>  6

ggagaacaga ggataaaagg ctc                                                23

<210>  7

<211>  20

<212>  DNA
<213>  Artificial Sequence

<220>
<221>  source
<223>  /note="Description of Artificial Sequence: Artificially synthesized
primer sequence for RT-PCR"

<400>  7
atcgccatct acaggagcag                                                    20

<210>  8
<211>  23
<212>  DNA
<213>  Artificial Sequence

<220>
<221>  source
<223>  /note="Description of Artificial Sequence: Artificially synthesized
primer sequence for RT-PCR"

<400>  8
ccaggcatgt ctttgtaggt aac                                                23

<210> 9

<211> 20

<212> DNA

<213> Artificial Sequence

<220>

<221> source

<223> /note="Description of Artificial Sequence: Artificially synthesized

primer sequence for RT-PCR"

<400> 9

accttcttag gcctcctggt 20

<210> 10

<211> 22

<212> DNA

<213> Artificial Sequence

<220>

<221> source

<223> /note="Description of Artificial Sequence: Artificially synthesized

primer sequence for RT-PCR"

<400> 10
   tacacgattg tcctcaccct tc 22

<210> 11

<211> 20

<212> DNA

<213> Artificial Sequence

<220>

<221> source

<223> /note="Description of Artificial Sequence: Artificially synthesized

primer sequence for RT-PCR"

<400> 11

ctggtatgcg actgggatct 20

<210> 12

<211> 21

<212> DNA

<213> Artificial Sequence

<220>

<221> source

<223> /note="Description of Artificial Sequence: Artificially synthesized

primer sequence for RT-PCR"

<400>  12

tgattgactc agcaatgcag g                                                21

<210>  13

<211>  20

<212>  DNA

<213>  Artificial Sequence

<220>

<221>  source

<223>  /note="Description of Artificial Sequence: Artificially synthesized

primer sequence for RT-PCR"

<400>  13

tcctgcactg actcctgaga                                                  20

<210>  14

<211>  23

<212>  DNA

<213>  Artificial Sequence

<220>

<221>  source

<223>  /note="Description of Artificial Sequence: Artificially synthesized

primer sequence for RT-PCR"

<400> 14
gaatagaaac agaaacccct tgg 23

<210> 15
<211> 20
<212> DNA
<213> Artificial Sequence

<220>
<221> source
<223> /note="Description of Artificial Sequence: Artificially synthesized
primer sequence for RT-PCR"

<400> 15
ccgaggtgga taacctgaca 20

<210> 16
<211> 23
<212> DNA
<213> Artificial Sequence

<220>

<221>  source
<223>  /note="Description of Artificial Sequence: Artificially synthesized
primer sequence for RT-PCR"

<400>  16
gaattggtcg aaaagtcctc aac                                              23

<210>  17
<211>  20
<212>  DNA
<213>  Artificial Sequence

<220>
<221>  source
<223>  /note="Description of Artificial Sequence: Artificially synthesized
primer sequence for RT-PCR"

<400>  17
ctcacacatt gcgaacaaca                                                  20

<210>  18
<211>  23
<212>  DNA
<213>  Artificial Sequence

<220>

<221> source

<223> /note="Description of Artificial Sequence: Artificially synthesized

primer sequence for RT-PCR"

<400> 18

atggtgcttt taagaagaga gcc 23

<210> 19

<211> 20

<212> DNA

<213> Artificial Sequence

<220>

<221> source

<223> /note="Description of Artificial Sequence: Artificially synthesized

primer sequence for RT-PCR"

<400> 19

gcctcacaaa agagccagag 20

<210> 20

<211> 23

<212>  DNA
<213>  Artificial Sequence

<220>
<221>  source
<223>  /note="Description of Artificial Sequence: Artificially synthesized
primer sequence for RT-PCR"

<400>  20
gatccacatt ggtgttacca gtt                                              23

<210>  21
<211>  20
<212>  DNA
<213>  Artificial Sequence

<220>
<221>  source
<223>  /note="Description of Artificial Sequence: Artificially synthesized
primer sequence for RT-PCR"

<400>  21
cgaccacttt gtcaagctca                                                  20

<210> 22

<211> 23

<212> DNA

<213> Artificial Sequence

<220>

<221> source

<223> /note="Description of Artificial Sequence: Artificially synthesized

primer sequence for RT-PCR"

<400> 22

ggttgagcac agggtacttt att 23

<210> 23

<211> 22

<212> DNA

<213> Artificial Sequence

<220>

<221> source

<223> /note="Description of Artificial Sequence: Artificially synthesized

primer sequence for RT-PCR"

<400> 23

tgacagcatg aggaaacaaa tc 22

<210> 24

<211> 20

<212> DNA

<213> Artificial Sequence

<220>

<221> source

<223> /note="Description of Artificial Sequence: Artificially synthesized

primer sequence for RT-PCR"

<400> 24

acgaccccaa gaaatgagtg 20

<210> 25

<211> 20

<212> DNA

<213> Artificial Sequence

<220>

<221> source

<223> /note="Description of Artificial Sequence: Artificially synthesized

primer sequence for RT-PCR"

<400> 25

gcattggtgg aggaggatac 20

<210> 26

<211> 24

<212> DNA

<213> Artificial Sequence

<220>

<221> source

<223> /note="Description of Artificial Sequence: Artificially synthesized

primer sequence for RT-PCR"

<400> 26

tgccattaac attctagcta ctgg 24

<210> 27

<211> 20

<212> DNA

<213> Artificial Sequence

<220>

<221> source

<223> /note="Description of Artificial sequence: Artificially synthesized

primer sequence for northern hybridization"

<400> 27
ggaaaattcg gaagaagcaa 20

<210> 28
<211> 20
<212> DNA
<213> Artificial Sequence

<220>
<221> source
<223> /note="Description of Artificial Sequence: Artificially synthesized
primer sequence for northern hybridization"

<400> 28
cgggatttgt tcagttcagg 20